(19) **Europäisches Patentamt**
**European Patent Office**
**Office européen des brevets**

(11) **EP 3 582 710 B1**

(12) **FASCICULE DE BREVET EUROPEEN**

(45) Date de publication et mention de la délivrance du brevet:
**28.02.2024 Bulletin 2024/09**

(21) Numéro de dépôt: **18711388.1**

(22) Date de dépôt: **20.02.2018**

(51) Classification Internationale des Brevets (IPC):
**A61B 34/20** (2016.01)   **A61B 90/00** (2016.01)
**A61B 17/17** (2006.01)

(52) Classification Coopérative des Brevets (CPC):
**A61B 34/20; A61B 90/39;** A61B 2034/2048;
A61B 2034/2055; A61B 2034/2068;
A61B 2090/067; A61B 2090/068; A61B 2090/0807;
A61B 2090/3983

(86) Numéro de dépôt international:
**PCT/FR2018/050390**

(87) Numéro de publication internationale:
**WO 2018/150151 (23.08.2018 Gazette 2018/34)**

(54) **SYSTÈME D'ORIENTATION CHIRURGICAL UTILISANT LA GÉOMÉTRIE DES OS POUR UN POSITIONNEMENT RÉPÉTABLE**

CHIRURGISCHES ORIENTIERUNGSSYSTEM MIT KNOCHENGEOMETRIE ZUR WIEDERHOLBAREN POSITIONIERUNG

SURGICAL ORIENTATION SYSTEM USING BONE GEOMETRY FOR REPEATABLE POSITIONING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.02.2017  FR 1751334**

(43) Date de publication de la demande:
**25.12.2019  Bulletin 2019/52**

(73) Titulaire: **Pytheas Navigation**
**13008 Marseille (FR)**

(72) Inventeurs:
• **POMERO, Vincent**
**13120 Gardanne (FR)**

• **GLARD, Yann**
**13008 Marseille (FR)**

(74) Mandataire: **Santarelli**
**Tour Trinity**
**1 bis Esplanade de la Défense**
**92035 Paris La Défense Cedex (FR)**

(56) Documents cités:
EP-A1- 1 088 525     FR-A1- 3 030 222
GB-A- 2 382 777      KR-A- 20060 003 685
US-A- 5 676 673      US-A1- 2011 257 653
US-A1- 2016 100 773

**Description**

<u>DOMAINE TECHNIQUE</u>

La présente invention concerne un système d'orientation chirurgical et un ancillaire utilisable dans le cadre d'un tel système.

<u>CONTEXTE DE L'INVENTION</u>

La structure squelettique interne d'un mammifère, humaine ou animale, est composée parfois d'une centaine d'os ou plus. La colonne vertébrale est une chaîne d'os ou de vertèbres permettant une certaine souplesse et liberté de mouvement, tout en protégeant les structures nerveuses et vasculaires à l'intérieur et autour de la colonne vertébrale. La colonne vertébrale commence à la base du crâne, s'étend jusqu'au bassin et est composée de quatre régions - cervicale, thoracique, lombaire et pelvienne.

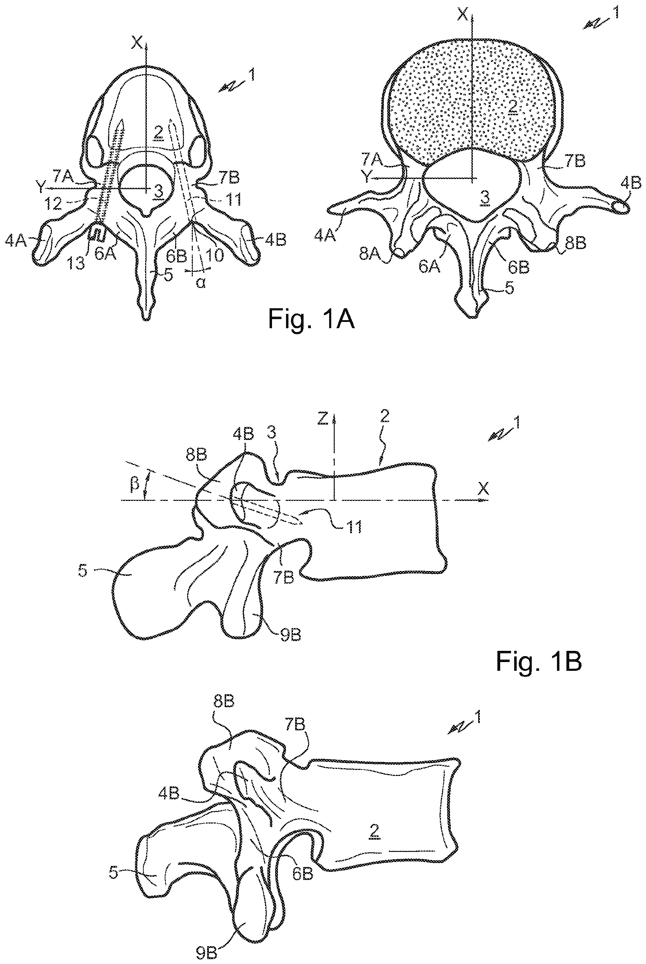

Les **figures 1A, 1B** représentent respectivement une vue de dessus et une vue de côté de vertèbres typiques humaine 1 (thoracique à gauche et lombaire à droite). La vertèbre 1 comprend : un corps vertébral 2 orienté vers l'avant ; un foramen vertébral 3 en forme de trou permettant à la moelle épinière de passer ; deux processus transversaux 4A, 4B, orientés vers l'arrière et vers l'extérieur ; un processus épineux 5 entre les processus transversaux 4A, 4B et orienté vers le bas ; deux lames 6A, 6B qui connectent les processus transversaux 4A, 4B au processus épineux 5 ; deux pédicules 7A, 7B qui connectent le corps vertébral 2 aux processus transversaux 4A, 4B ; deux facettes articulaires supérieures 8A (non représenté), 8B et deux facettes articulaires inférieures 9A (non représenté), 9B qui permettent l'articulation des vertèbres 1 entre-elles.

L'alignement vertébral normal ou idéal peut être perturbé suite à un traumatisme ou une maladie, par exemple la scoliose. Les vertèbres peuvent pivoter autour de trois axes (X, Y, Z), nécessitant parfois une intervention chirurgicale afin de corriger les anomalies et retrouver un alignement idéal ou, à tout le moins amélioré, de la colonne vertébrale.

Dans ce cas, au moins deux vertèbres 1 adjacentes sont généralement fusionnées l'une à l'autre par un procédé dans lequel un chirurgien opère le patient, généralement par le dos, détermine un point d'entrée 10 et perce des trous 11 dans les pédicules 7A, 7B des vertèbres 1. Les trous sont percés avec un angle axial alpha $\alpha$ (l'angle par rapport au plan XZ) et un angle sagittal beta $\beta$ (l'angle par rapport au plan XY), montrés aux **figures 1A, 1B** respectivement.

Ensuite, des vis pédiculaires 12 comprenant des extrémités 13 en forme de U sont insérées dans les trous 11 (Pour des raisons de clarté de la **figure 1A,** un seul point d'entrée 10, trou 11, vis pédiculaire 12, et extrémité 13 sont montrés). Les extrémités 13 reçoivent des éléments de liaison (non montrés), par exemple des barres, qui permettent de réduire la déformation et de fusionner les vertèbres 1 entre elles.

Ensuite, les éléments de liaison sont connectés entre les vis pédiculaires de deux vertèbres 1 adjacentes afin de corriger, au fur et à mesure, l'alignement de la colonne vertébrale, qui est basé sur des objectifs de correction approximatifs pour chaque niveau de la colonne vertébrale et dérivés à partir d'images médicales (rayons électromagnétiques 'X', tomodensitométrie, imagerie par résonnance magnétique, etc.) prises pré-opératoirement, c'est-à-dire avant une intervention chirurgicale. En conséquence, tous les trous 11 percés et les vis pédiculaires 12 placées dans les vertèbres 1 doivent être soigneusement positionnés et alignés afin de ne pas blesser les structures nerveuses et vasculaires adjacentes. Dans le cas où la vis pédiculaire 12 serait mal positionnée et entrainerait un risque lésionnel pour les nerfs, la moelle épinière ou les vaisseaux sanguins, il faudrait procéder à une seconde opération, entraînant des coûts et des risques supplémentaires.

Des systèmes de guidage ont été développés pour aider le chirurgien à percer les trous 11 dans la vertèbre 1 et placer précisément les vis pédiculaires 12. Par exemple, la publication WO 2016/102898 décrit des outils, accessoires, ou ancillaires chirurgicaux, pour indiquer au préalable le bon trajet au chirurgien par rapport aux images prises pré-opératoirement pour tenir compte de la position du patient pendant l'opération.

Selon cette publication, un ancillaire chirurgical comprend un premier point de contact destinée à venir en contact avec un premier point de référence d'une zone opératoire ; un deuxième point de contact destinée à venir en contact avec un deuxième point de référence de la zone opératoire ; et une zone de contact destinée à venir en contact avec un troisième point de référence de la zone opératoire ;

L'ancillaire comprend en outre un moyen de détermination d'un référentiel d'orientation de l'ancillaire dans un référentiel d'orientation galiléen ; et un moyen de communication du référentiel d'orientation déterminé par son positionnement adéquat sur la vertèbre.

Par exemple, un ancillaire en forme de Y est proposé dans lequel les premier et deuxième points de contact sont réalisés à l'aide d'arêtes rectilignes pourvues aux extrémités de deux branches du Y. Les deux points de contact sont posés sur les deux premiers points de référence correspondants avant de faire pivoter l'ancillaire autour de l'axe formé par les deux points de contact pour venir poser la zone de contact sur le troisième point de référence.

D'une façon plus générale, un tel ancillaire permet de guider tout opérateur réalisant une intervention, non nécessairement chirurgicale, sur une zone d'intervention, par exemple sur une pièce mécanique dont on souhaite connaître l'orientation précise dans un référentiel galiléen.

Bien que les arêtes formant points de contact puissent être dotées de « picots » antiglissement, le pivotement de l'ancillaire en conservant les deux points de contact posés sur les points de référence correspondant s'avère périlleux et requiert une grande dextérité de la part de l'opérateur ou du chirurgien. En outre, le positionnement de l'ancillaire est difficilement réalisé sur les deux points de contact exactement tels qu'initialement déterminé.

Il en résulte un défaut de répétabilité, pour l'opérateur, du positionnement exact de l'ancillaire sur les points de référence. Ce défaut peut entraîner une estimation erronée du référentiel de la vertèbre, dommageable à la réalisation précise de l'acte chirurgical ou de l'ouvrage mécanique.

En conséquence, il existe un besoin de systèmes et outils simples d'aide à l'opérateur ou au chirurgien qui permette un positionnement plus précis et moins dépendant de l'opérateur de l'ancillaire pour déterminer, avec une meilleure répétabilité, l'orientation de la vertèbre pendant la chirurgie.

RESUME DE L'INVENTION

La présente invention vise à résoudre les limitations ci-dessus des outils connus. La présente invention est définie par la revendication 1.

Dans ce contexte, des modes de réalisation de l'invention concernent un ancillaire chirurgical en forme de i-grec (y) comprenant au moins :

- une première partie de contact destinée à venir en contact avec une première zone de référence d'une zone opératoire ;
- une deuxième partie de contact destinée à venir en contact avec une deuxième zone de référence de la zone opératoire ;
- au moins deux branches aux extrémités desquelles les première et deuxième parties de contact sont disposées;
- une troisième partie de contact destinée à venir en contact avec une troisième zone de référence de la zone opératoire ;
- une troisième branche en forme de poignée
- une zone centrale ayant une face inférieure qui forme la troisième partie de contact; et
- un moyen de détermination d'un référentiel d'orientation de l'ancillaire dans un référentiel d'orientation galiléen.

Dans cet ancillaire chirurgical, au moins une des première et deuxième parties de contact comporte une extrémité concave de section courbe ou convexe, destinée à venir en contact avec au moins deux points de référence dans la première ou deuxième zone de référence correspondante qui est de forme convexe ou concave respectivement (c'est-à-dire de forme sensiblement complémentaire à l'extrémité considérée).

A titre d'exemple, l'ancillaire peut être en forme de Y doté d'une poignée et de deux cylindres aux extrémités de deux des branches, de sorte à ce que chaque cylindre vienne se loger dans une zone concave de la vertèbre, par exemple les lames. Le positionnement des cylindres dans les zones concaves se fait naturellement compte tenu de leurs géométries respectives. Les points de contact sont alors ceux effectivement prévus initialement.

Un autre exemple est un ancillaire en forme de Y doté d'une poignée et de deux éléments en forme de gouttière aux extrémités de deux des branches, de sorte à ce que chaque élément en forme de gouttière vienne envelopper une zone convexe de la vertèbre, par exemple les processus transversaux.

On comprend que les points de contact sont réalisés au niveau de la surface concave/convexe du cylindre ou de l'élément en forme de gouttière (et non sur une arête délimitant une extrémité de ceux-ci).

Comme montré par ces deux exemples, la présente invention tire parti de la concavité/convexité des géométries osseuses pour créer, à l'aide de l'extrémité convexe/concave, un positionnement répétable de l'ancillaire et un guide de rotation de celui-ci vers la position assurant le contact de la troisième partie de contact. En effet, les points de contact générés par les deux extrémités permettent par exemple de définir à tout le moins une liaison pivot entre l'ancillaire et la zone opératoire.

La rotation entre partie convexe et partie concave correspondante (selon les deux points de contact indiqués) sécurise la rotation de l'ancillaire. L'opérateur peut ainsi se concentrer sur la mise en contact de l'ancillaire au niveau de la troisième zone de contact.

Une plus grande précision de positionnement, une meilleure reproductibilité des opérations et une réduction du temps de manipulation de l'ancillaire sont ainsi obtenues.

Des caractéristiques optionnelles de l'ancillaire chirurgical sont par ailleurs définies dans les revendications dépendantes.

Selon un mode de réalisation, les première et deuxième parties de contact comportent chacune une extrémité concave ou convexe destinée à venir en contact avec au moins deux points de référence de la zone de référence convexe ou concave correspondante. Cette disposition sécurise encore plus le positionnement de l'ancillaire, tout en libérant l'opérateur d'une vérification du contact permanent des deux premières parties contact avec la zone opératoire. En effet, un double guidage par rotation est ainsi obtenu.

Selon un mode de réalisation, les deux extrémités sont de section courbe avec des axes générateurs sensiblement coaxiaux. Les deux extrémités peuvent être toutes deux concaves, toutes deux convexes ou d'un type différent.

Cette disposition facilite la rotation, et donc le basculement/pivotement, de l'ancillaire dans la recherche d'un contact de la troisième partie de contact avec la zone opératoire. En effet, une rotation de l'ancillaire selon cet axe commun, par les deux zones de guidage, est ainsi obtenue.

Selon un autre mode de réalisation, les deux extrémités présentent des sections transversales courbes (concaves ou convexes) et présentent des sections longitudinales qui appartiennent à une même courbe conique. Les exemples les plus classiques de courbes coniques sont le cercle, l'ellipse ou encore la parabole.

Cette disposition facilite la rotation de l'ancillaire dans un plan contenant l'ancillaire (dans sa réalisation sensiblement plane comme illustrée par la suite). Cette facilité de rotation peut par exemple venir compenser un défaut de basculement/pivotement à raison de contacts indésirables avec la zone opératoire.

Selon un mode de réalisation, l'autre partie de contact parmi la première et deuxième parties de contact est formée d'une arête rectiligne ou d'un pointeau destiné à venir en contact avec un unique point de référence de la zone de référence correspondante. Cette configuration permet de s'adapter à une asymétrie anatomique (soit résultante d'une malformation, soit à raison de la zone opératoire asymétrique considérée).

Selon un mode de réalisation, l'extrémité convexe comporte un élément en forme de rouleau, galet, cylindre ou sphère (c'est-à-dire une forme convexe sensiblement régulière aux incertitudes de fabrication près) destiné à s'engager dans la zone de référence concave correspondante, ou l'extrémité concave comporte un élément en forme de gouttière (typiquement une paroi d'extrémité semi-cylindrique ou sensiblement semi-cylindrique, c'est-à-dire un cylindre creux tronqué longitudinalement) ou de coupelle sphérique (forme également sensiblement régulière), destiné à s'engager sur la zone de référence convexe correspondante.

Ces formes régulières permettent notamment une rotation régulière, sans modification des deux points de contact au moins (trois points de contact pour une sphère par exemple) pour chaque extrémité. Le confort de manipulation pour l'opérateur est donc amélioré. Préférentiellement, les deux extrémités sont formées d'éléments coaxiaux, assurant cette rotation régulière selon le même axe.

Selon un mode de réalisation, les première et deuxième parties de contact comportent des éléments convexes ou concaves de même forme (mais potentiellement de dimensions différentes pour répondre à des variations anatomiques) à leur extrémité pour venir en contact avec leur zone de référence respective.

Cela contribue à une rotation ou à un basculement régulier de l'ancillaire, pour l'opérateur.

Bien entendu, en variante, des éléments de formes différentes peuvent être utilisés pour s'adapter aux anatomies asymétriques, comme déjà évoqué ci-dessus.

Selon un mode de réalisation, l'élément formant extrémité convexe ou concave est monté mobile en rotation sur l'ancillaire. Il peut s'agir par exemple d'un rouleau ou cylindre rotatif, ou d'un profil de type gouttière rotatif

Cette disposition permet de conserver inchangés les deux points de contact pour chaque extrémité convexe/concave, notamment en cas de rotation autour de l'axe de révolution (ou axe générateur) de cette extrémité. On évite ainsi tout risque de glissement ou dérapage des points de contact déjà établis lors du basculement. Le confort de manipulation pour l'opérateur est donc encore amélioré.

Selon un mode de réalisation, l'extrémité convexe ou concave présente un rayon de courbure supérieur à un rayon de courbure local (c'est-à-dire dans la zone de contact) de la zone de référence concave ou convexe correspondante.

Cette disposition contribue à ce qu'il n'y ait que deux points de contact entre l'extrémité et la zone opératoire en regard, compte tenu des irrégularités anatomiques de cette dernière. Le guidage en rotation et donc la facilité de manipulation sont améliorés.

Selon un mode de réalisation, la troisième partie de contact est destinée à venir en contact avec la troisième zone de référence par basculement de l'ancillaire alors que les première et deuxième parties de contact sont en contact avec les première et deuxième zones de référence respectivement. Notamment la partie de contact concave ou convexe réalise un contact en deux points avec la zone de référence correspondante.

Selon un mode de réalisation, l'extrémité concave ou convexe comporte une surface de section courbe autour d'un axe de révolution, et le basculement s'opère autour de l'axe de révolution, par exemple l'axe de rotation d'un élément formant extrémité convexe ou concave monté mobile en rotation sur l'ancillaire. Préférentiellement, les extrémités formant première et deuxième parties de contact sont du même type, avec des axes de révolution colinéaires, pour permettre une rotation aisée.

Selon un mode de réalisation, la première partie de contact, la deuxième partie de contact, la troisième partie de contact, le moyen de détermination et éventuellement un moyen de communication discuté par la suite sont intégrés dans une seule partie de l'ancillaire.

Selon un mode de réalisation, la première partie de contact, la deuxième partie de contact et la troisième partie de contact sont intégrées dans une première partie de l'ancillaire, et le moyen de détermination et éventuellement le moyen de communication sont intégrés dans une deuxième partie apte à être encastrée sur la première partie, c'est-à-dire à être rendue solidaire de la première partie de l'ancillaire à l'aide de moyens mécaniques.

Selon un mode de réalisation, la troisième partie de contact est une zone de contact tangentiel essentiellement plane.

Selon un mode de réalisation, la troisième partie de contact est formée d'une zone concave ou convexe destinée à venir en contact avec au moins deux points de référence de la troisième zone de référence qui est de forme convexe ou concave respectivement. Cette disposition facilite grandement le positionnement exact de l'ancillaire sur la zone opératoire, compte tenu des points de référence (déterminés par exemple de façon préopératoire par ordinateur). L'obtention du référentiel d'orientation avec positionnement exact est ainsi fortement reproductible, ces deux points de contacts dans la troisième partie de contact permettant de lever l'incertitude du positionnement de l'ancillaire défini par l'axe de rotation créé par les points de contact des premières et deuxièmes parties de contact.

Selon un mode de réalisation, la troisième partie de contact est formée de deux surfaces planes, destinées chacune à venir en contact avec un unique point de référence dans la troisième zone de référence. Cette configuration est simple à mettre en oeuvre au niveau de la troisième partie de contact. En outre, une surface plane facilite le glissement de l'ancillaire le long de l'unique point de référence (de contact) correspondant, pour positionner l'autre surface en contact avec l'autre unique point de référence. Ainsi, le positionnement de l'ancillaire est totalement contrôlé et reproductible.

Selon un mode de réalisation, la troisième partie de contact présente une forme en V ou en V inversé, selon que la troisième zone de référence est concave ou convexe. Bien entendu des variantes de forme peuvent être prévues pour autant qu'elles permettent un contact en deux points de référence entre les troisièmes parties de contact et de référence : une gorge de toute forme, par exemple parabolique ou parabolique inversée.

Selon un mode de réalisation, les première et deuxième parties de contact et l'une des deux surfaces planes de la troisième partie de contact font partie d'une même pièce monobloc, et l'autre surface plane de la troisième partie de contact est constituée d'une pièce mécanique rapportée (de préférence de façon solidaire) sur la pièce monobloc. Cela permet de concevoir aisément cette troisième partie de contact (permettant un positionnement précis) à partir d'une surface plane, telle que proposée dans les ancillaires connus.

Selon un mode de réalisation, le référentiel d'orientation de l'ancillaire permet de déterminer un référentiel d'orientation de la zone opératoire par rapport au référentiel d'orientation galiléen au moyen par exemple d'une matrice de rotation.

Selon un mode de réalisation, l'ancillaire comprend en outre des moyens de validation de la troisième partie de contact.

Selon un mode de réalisation, la troisième partie de contact est transparente et agencée d'une forme quadrillée et marquée afin de permettre à un opérateur de déterminer où exactement la troisième partie de contact est en contact avec un point de la troisième partie de référence. Cela permet de déterminer complètement la position et l'orientation de l'ancillaire.

Selon un mode de réalisation, on peut ajuster la longueur, l'angle, et/ou l'inclinaison d'au moins une branche de l'ancillaire.

Selon un mode de réalisation, le moyen de détermination du référentiel est un dispositif comprenant au moins l'un des composants suivants : un accéléromètre tri-axe ; un magnétomètre tri-axe ; et/ou un gyroscope tri-axe.

Selon un mode de réalisation, le moyen de détermination du référentiel d'orientation est un dispositif comprenant au moins trois marqueurs optiques non alignés, destinés à être visible par au moins une caméra filmant la zone opératoire.

Selon un mode de réalisation, l'ancillaire comprend en outre un moyen de communication du référentiel d'orientation déterminé.

Selon un mode de réalisation, le moyen de communication du référentiel d'orientation est un affichage visuel.

Selon un mode de réalisation, le moyen de communication du référentiel d'orientation est une liaison filaire ou non-filaire.

Des modes de réalisation de l'invention concernent en outre un ensemble comprenant au moins deux ancillaires différents selon l'invention, les ancillaires étant conçus pour des zones opératoires différentes l'une de l'autre.

Des modes de réalisation de l'invention concernent en outre un système d'orientation chirurgical comprenant au moins un ancillaire selon l'invention et un outil chirurgical comprenant :

- un moyen de détermination d'un référentiel d'orientation de l'outil ; et

- un moyen de communication du référentiel d'orientation de l'outil.

Selon un mode de réalisation, l'ancillaire et l'outil chirurgical sont configurés pour être couplés à un même dispositif de détermination et de communication de référentiel d'orientation.

Des modes de réalisation de l'invention concernent en outre une salle opératoire équipée d'un ancillaire chirurgical selon l'invention et d'un dispositif d'affichage d'images et de traitement de données comprenant :

- un écran ou un moyen de projection pour afficher des images prises de la zone opératoire ;
- un processeur ;
- des moyens d'entrée et de manipulation de données ; et
- des moyens de réception des données communiquées par l'ancillaire.

Des exemples ne faisant pas partie de l'invention concernent en outre un procédé de préparation préopératoire d'une opération chirurgicale, comprenant les étapes consistant à :

- prendre au moins une image tridimensionnelle d'une zone opératoire ;
- déterminer au moins deux points de référence dans une première zone de référence de la zone opératoire, et au moins deux autres points de référence d'une deuxième zone de référence (potentiellement deux autres paires de points pour les deuxième et troisième zones de référence) de la zone opératoire, à partir de l'image tridimensionnelle. En pratique, on peut envisager qu'un opérateur indique uniquement des zones anatomiques correspondant aux zones de référence, et qu'un algorithme informatique détermine les points de référence dans ces zones compte tenu des dimensions géométriques de l'ancillaire à utiliser ;
- calculer un référentiel opératoire au moyen des points de référence, le référentiel opératoire étant identifiable ultérieurement par un ancillaire selon l'invention ; et
- déterminer au moins un axe local dans le référentiel opératoire, pour un geste chirurgical à réaliser.

D'autres exemples ne faisant pas partie de l'invention visant à proposer des systèmes et outils simples d'aide à l'opérateur (non chirurgien) ou au chirurgien concernent un ancillaire ou outil en forme de compas à deux branches. L'ancillaire comprend au moins :

- une première branche ayant une première extrémité terminale de contact destinée à venir en contact avec une première zone de référence d'une zone d'intervention ;
- une deuxième branche ayant une deuxième extrémité terminale de contact destinée à venir en contact avec une deuxième zone de référence de la zone d'intervention ;
- une partie fixe relativement à laquelle les deux branches se déplacent pour ouvrir ou fermer l'ancillaire, de sorte que, lors de l'ouverture ou de la fermeture de l'ancillaire, les première et deuxième extrémités terminales forment un axe constamment parallèle à un axe de référence de la partie fixe ; et
- un moyen de détermination d'une orientation de l'ancillaire. Il s'agit préférentiellement de l'orientation de l'axe formé par les première et deuxième extrémités.

Comme évoqué par la suite, le moyen de détermination d'une orientation de l'ancillaire, typiquement une centrale inertielle, permet de déterminer deux orientations lors de deux mesures, et ainsi déterminer un référentiel d'orientation de l'ancillaire (référentiel d'intervention) dans un référentiel galiléen.

Cet outil est simple à manipuler pour un opérateur ou un chirurgien. En outre, la propriété géométrique des extrémités terminales (formant un axe constamment parallèle) permet d'avoir recours à un seul moyen de détermination d'une orientation pour la détermination d'un référentiel d'intervention de la zone d'intervention, au sein d'un référentiel d'orientation galiléen. On s'affranchit ainsi de tout besoin de calibration relative de deux capteurs qui seraient montés sur chacune des branches.

A cet effet, ces exemples ne faisant pas partie de l'invention prévoient également un procédé de détermination *in situ* d'un référentiel d'intervention d'une zone d'intervention, dans un référentiel d'orientation galiléen, à l'aide d'un ancillaire de type compas à deux branches équipé d'un moyen de détermination d'une orientation de l'ancillaire, les deux branches de l'ancillaire se déplaçant, lors de l'ouverture ou de la fermeture de l'ancillaire, de sorte que leurs extrémités terminales forment un axe constamment parallèle à un axe de référence d'une partie fixe de l'ancillaire. Le procédé comprend les étapes consistant à :

- obtenir, du moyen de détermination d'une orientation, au moins deux orientations non parallèles dans le référentiel d'orientation galiléen entre deux paires de zones de référence au sein de la zone d'intervention, en positionnant successivement les deux extrémités terminales de l'ancillaire sur chacune des deux paires de zones de référence ; et
- déterminer le référentiel d'intervention à partir des deux orientations obtenues.

Ainsi, l'opérateur peut déterminer un référentiel d'intervention, au sein du référentiel galiléen, par des opérations simples et peu nombreuses. La mesure de seulement deux orientations au travers de deux positionnements successifs uniquement du compas est suffisante.

Le procédé s'applique avantageusement à la détermination de référentiels de pièces mécaniques sur lesquelles des interventions nécessitant de la précision sont prévues, par exemple pour y réaliser des perçages ou placer des moyens de fixation.

A titre d'exemple, un plan du référentiel d'intervention peut être formé par le plan défini par les deux orientations obtenues. En outre, un axe du référentiel d'intervention peut être selon l'une des orientations obtenues, par exemple la première.

Ainsi, il est également prévu un procédé de guidage pour une intervention sur une zone d'intervention, comprenant les étapes consistant à :

- obtenir au moins trois zones de référence dans la zone d'intervention ;
- obtenir un référentiel d'intervention au moyen des zones de référence ;
- déterminer au moins un axe d'intervention dans le référentiel d'intervention, pour un geste d'intervention à réaliser ;
- déterminer *in situ* ledit référentiel d'intervention de la zone d'intervention dans un référentiel d'orientation galiléen, à l'aide du procédé ci-dessus ; et
- réaliser un geste d'intervention sur la zone d'intervention à l'aide d'un outil d'intervention, au moins un axe de l'outil d'intervention étant déterminé dans le référentiel d'orientation galiléen et étant mis en correspondance avec l'axe d'intervention dans le référentiel d'intervention.

Cette correspondance peut par exemple être effectuée par conversion d'au moins un desdits axes d'un référentiel à l'autre. Bien entendu, plusieurs axes d'intervention (2 ou 3) peuvent être déterminés initialement afin de les faire correspondre exactement aux axes correspondants de l'outil d'intervention. Un tel contrôle de l'orientation de l'outil peut s'avérer important lorsque l'on souhaite maîtriser le positionnement et l'orientation d'une pièce à fixer dans la zone d'intervention. A titre illustratif seulement, ce peut être le cas du positionnement sensible d'un implant dans une zone anatomique.

La conversion peut simplement comprendre l'application d'une matrice de rotation sur un vecteur définissant soit l'axe d'intervention, soit l'axe de l'outil d'intervention.

Différentes versions de l'ancillaire de type compas peuvent être utilisées.

Par exemple, les première et deuxième branches peuvent être montées mobiles en rotation sur la partie fixe et être agencées pour s'ouvrir ou se fermer de façon symétrique relativement à la partie fixe. Une telle version de l'ancillaire est facilement utilisable par un opérateur. Préférentiellement, le moyen de détermination d'une orientation est de type centrale inertielle montée solidaire de la partie fixe. La centrale inertielle, fixe par rapport à l'axe de référence, permet ainsi de connaître, sans calcul ou moyen additionnel, l'orientation entre deux zones de référence au contact des deux extrémités terminales du compas.

Selon une variante, les première et deuxième branches sont montées mobiles en translation par rapport à la partie fixe et sont agencées pour coulisser en translation, éventuellement de façon symétrique, relativement à la partie fixe. Dans ce cas, le moyen de détermination d'une orientation peut être de type centrale inertielle montée solidaire de la première ou deuxième branche, ou être montée solidaire de la partie fixe.

Dans un exemple, au moins l'une des deux extrémités terminales, voire les deux, est formée d'une pointe de contact destinée à venir en contact avec un point de référence de la zone de référence correspondante. Ainsi, pour ce qui concerne le procédé de détermination *in situ* d'un référentiel d'intervention, deux paires de zones de références comprennent au moins trois points de référence non alignés, et les extrémités terminales des branches de l'ancillaire sont formées de pointes destinées à venir en contact avec les points de référence.

En variante, au moins l'une des deux extrémités terminales, voire les deux, est formée d'une extrémité concave ou convexe destinée à venir en contact avec au moins deux points de référence de la première ou deuxième zone de référence correspondante qui est de forme convexe ou concave respectivement. A noter que dans ce cas, l'axe qui reste constamment parallèle peut être défini par les centres des extrémités concaves/convexes (par exemple un point central sur l'axe d'un cylindre ou d'un rouleau, ou le centre d'une sphère). Les différentes configurations évoquées dans les exemples ci-dessus sont applicables aux extrémités de l'ancillaire de type compas défini ici.

Dans un exemple, le moyen de détermination d'une orientation comprend au moins l'un des composants suivants : un accéléromètre tri-axe ; un gyroscope tri-axe; et/ou un magnétomètre tri-axe.

Dans un exemple, l'obtention des trois zones de référence, du référentiel d'intervention et la détermination de l'axe d'intervention sont réalisées sur un modèle numérique tridimensionnel de la zone d'intervention. Cette disposition permet de tirer profit des capacités calculatoires des systèmes informatiques.

Dans un exemple qui vise à simplifier l'intervention de l'opérateur, réaliser le geste d'intervention comprend les étapes suivantes :

- afficher, sur un écran, un modèle numérique tridimensionnel de la zone d'intervention, ledit affichage étant préférentiellement (mais non nécessairement) réalisé selon l'axe d'intervention ;

- afficher, sur l'écran, un indicateur d'un point d'intervention sur la zone d'intervention et un indicateur d'orientation, dans le référentiel d'affichage, de l'outil d'intervention positionné sur le point d'intervention ; et
- réaliser le geste d'intervention sur la zone d'intervention lorsque les deux indicateurs sont confondus.

A noter que des indicateurs supplémentaires (par exemple de rotation) peuvent permettent de mettre en correspondance un deuxième axe de l'outil d'intervention (premier indicateur supplémentaire) avec un deuxième axe d'intervention (deuxième indicateur supplémentaire).

Des exemples ne faisant pas partie de l'invention concernent en outre un support non transitoire lisible par un ordinateur et comprenant une programme d'instructions exécutable par ordinateur pour effecteur l'un des procédés selon l'invention.

## BREVE DESCRIPTION DES FIGURES

D'autres caractéristiques et avantages particuliers de la présente invention ressortiront de la description détaillée faite en référence aux figures dans lesquelles :

- les **figures 1A, 1B,** précédemment décrites, représentent respectivement une vue de dessus et une vue de côté de vertèbres thoracique et lombaire typiques,
- la **figure 2A** représente une vue de dessus de deux systèmes d'orientation chirurgicaux comprenant chacun un ancillaire chirurgical et un outil chirurgical selon un mode de réalisation,
- la **figure 2B** représente deux vertèbres thoracique et lombaire avec des zones de référence pour l'opération,
- les **figures 3A, 3B** représentent respectivement une vue de dessus et une vue de côté des ancillaires chirurgicaux montrés à la **figure 2A** en cours d'utilisation sur des vertèbres thoracique et lombaire,
- la **figure 4** représente un référentiel d'orientation d'une salle opératoire et un référentiel d'orientation d'une zone opératoire,
- la **figure 5** représente schématiquement un système d'orientation chirurgical selon un autre mode de réalisation,
- la **figure 6** représente schématiquement un système d'orientation chirurgical selon un autre mode de réalisation,
- la **figure 7** représente schématiquement un système d'orientation chirurgical selon un autre mode de réalisation,
- les **figures 8A, 8B** représentent respectivement une vue en perspective d'un système d'orientation selon deux autres modes de réalisation et une vue en coupe, au niveau du processus épineux, de leur utilisation sur des vertèbres thoracique et lombaire,
- la **figure 9** représente une salle opératoire équipée d'un système d'orientation chirurgical selon un mode de réalisation,
- la **figure 10** représente un organigramme d'une phase préopératoire,
- la **figure 11** représente un organigramme d'une phase peropératoire,
- la **figure 12** représente un support non transitoire lisible par un ordinateur et comprenant une programme d'instructions exécutable par ordinateur,
- les **figures 13A** et **13B** représentent des vues en perspective d'un système d'orientation simplifié, dans deux positions différentes de mesure,
- les **figures 14A** et **14B** représentent des vues en perspective d'une variante de système d'orientation simplifié, dans deux positions différentes de mesure,
- la **figure 14C** représente une vue du dessus du système des **figures 14A** et **14B,**
- la **figure 15** représente un organigramme d'une phase préalable à une intervention sur une zone d'intervention,
- la **figure 16** représente un organigramme d'une phase opérationnelle d'intervention, et
- la **figure 17** illustre un affichage d'aide au guidage d'un opérateur lors d'une intervention.

## DESCRIPTION DETAILLEE DE MODES DE REALISATION

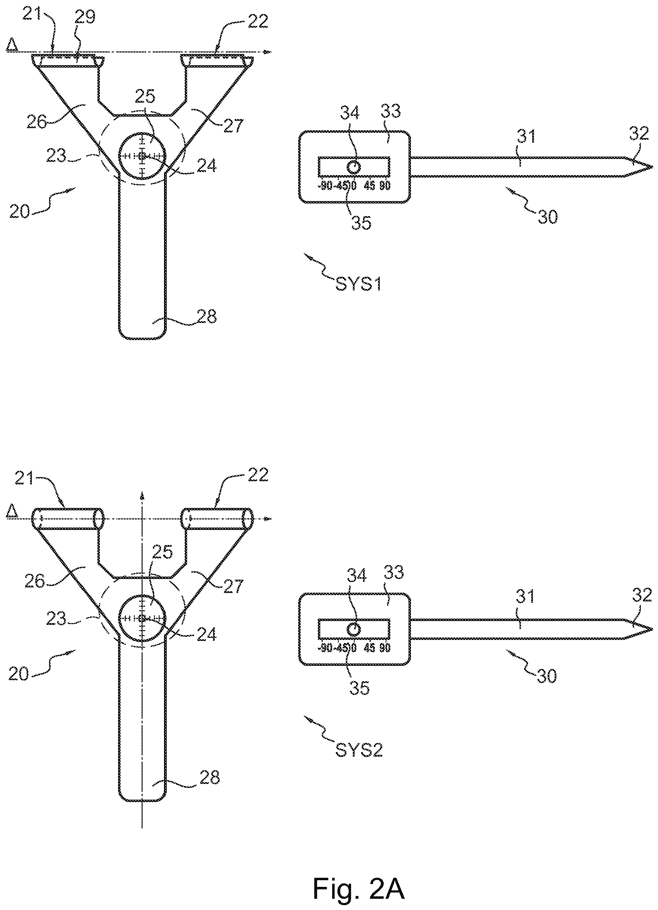

La **figure 2A** représente deux systèmes d'orientation chirurgicaux SYS1, SYS2 selon des modes de réalisation.

Le système SYS1 comprend un outil 20 (ci-après « ancillaire ») et un outil chirurgical 30, par exemple de forage.

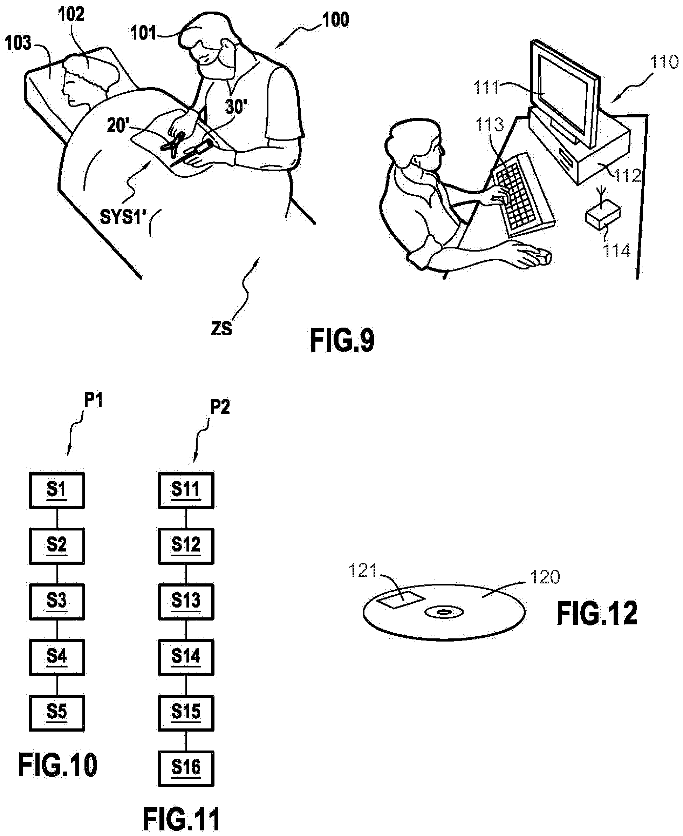

L'ancillaire 20 comprend : au moins deux parties de contact 21, 22 ; une troisième partie de contact, ici une zone plane de contact tangentiel 23 ; un moyen de détermination 24 d'un référentiel d'orientation RA de l'ancillaire et un moyen de communication 25 du référentiel d'orientation RA de l'ancillaire. Le référentiel d'orientation RA de l'ancillaire 20 peut être assimilé à un référentiel d'orientation RO d'une zone opératoire ZO (par exemple la vertèbre 1) et permet en conséquence de connaître le référentiel d'orientation RO dans un référentiel d'orientation galiléen RS, par exemple d'une salle opératoire ZS (montrée à la **figure 9**). Dans ce qui suit, le terme référentiel d'orientation RO de la zone opératoire sera utilisé.

Dans ce mode de réalisation, l'ancillaire 20 est en forme de Y (« i-grec »), les parties de contact 21, 22 étant des éléments concaves, notamment des éléments coaxiaux en forme de gouttières (c'est-à-dire des parois semi-cylindriques) positionnés aux extrémités d'une première branche 26 et d'une deuxième branche 27 respectivement, et une troisième branche 28 servant de poignée. Les branches 26, 27 sont les extrémités supérieures gauche et droite de l'Y respectivement, la branche 28 est l'extrémité inférieure centrale de l'Y, et la troisième partie de contact 23 est disposée au centre de l'Y et comprend une face inférieure plane (plus ou moins). La paroi interne 29 des éléments concaves 21, 22 est de préférence lisse afin de permettre un glissement de points de référence de la zone opératoire lors d'une manipulation de l'ancillaire 20 comme expliqué par la suite.

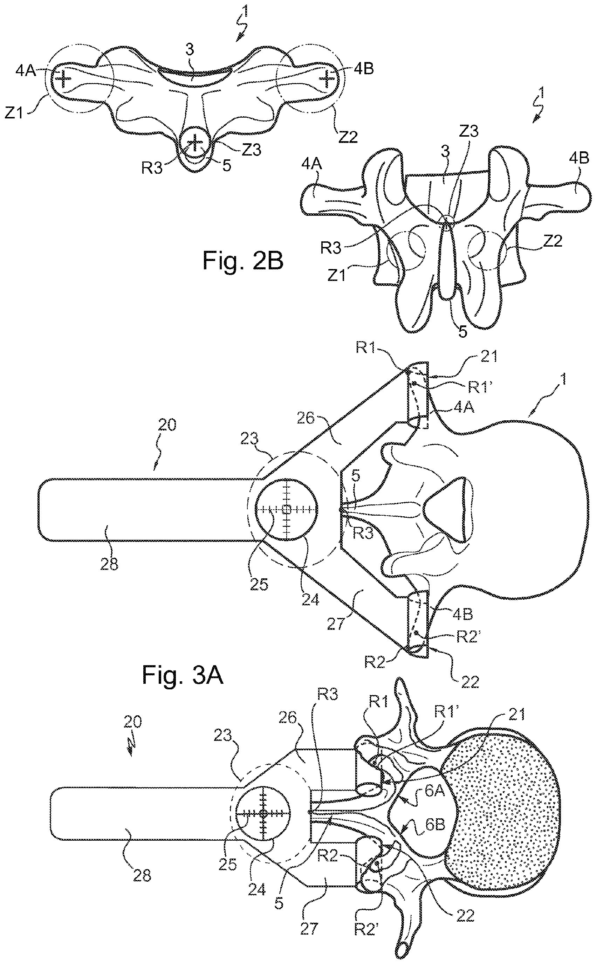

Comme montrée sur la partie de gauche de la **figure 2B,** la zone opératoire ZO (ici une vertèbre thoracique 1) comprend au moins trois zones de référence Z1, Z2, Z3 à utiliser avec le système SYS1. Dans le cas d'une vertèbre thoracique 1, les zones Z1, Z2 sont constituées par exemple des parois postérieures des processus transversaux 4A, 4B respectivement (parois convexes orientées vers le bas de la **figure 1A**), et sont très faciles à repérer à l'œil nu, en particulier par un chirurgien expérimenté. La zone de contact Z3, ici simplifiée à un unique point R3, est disposée sur le processus épineux 5 dans une zone tangentielle, comme cela sera expliqué plus loin. En conséquence, les parties de contact concaves 21, 22 sont chacune destinées à venir en contact avec les zones de références convexes Z1, Z2 respectivement et la troisième partie de contact 23 est posée sur le point R3 afin de déterminer un système de coordonnées ou « référentiel d'orientation » RO de la zone opératoire ZO par rapport au référentiel d'orientation RS de la salle opératoire ZS.

Le système SYS2 comprend également un outil 20 (ci-après « ancillaire ») et un outil chirurgical 30, par exemple de forage.

Comparativement au SYS1, le système SYS2 comporte un ancillaire 20 en forme de Y dont les parties de contact 21, 22 sont des éléments convexes, notamment des éléments coaxiaux en forme de rouleau, cylindre ou galet (c'est-à-dire des parois sensiblement cylindriques non nécessairement fermées) positionnés aux extrémités des branches 26, 27 respectivement.

Les éléments convexes 21, 22 peuvent être lisses afin de permettre un glissement de points de références de la zone opératoire lors d'une manipulation de l'ancillaire 20 comme expliqué par la suite. En variante, ces éléments convexes 21, 22 peuvent être montés mobile en rotation sur l'ancillaire, de préférence selon le même axe Δ, auquel cas leurs surfaces peuvent être pourvues d'un revêtement antidérapant afin d'assurer un contact durable avec des points des zones de référence correspondantes Z1, Z2.

Sur la partie de droite de la **figure 2B,** une zone opératoire ZO (ici une vertèbre lombaire 1) comprend à nouveau au moins trois zones de référence Z1, Z2, Z3 à utiliser avec le système SYS2. Dans le cas de cette vertèbre lombaire 1, les zones Z1, Z2 sont constituées par exemple des lames 6A, 6B respectivement (formant des cavités concaves sous les processus transversaux), et sont très faciles à repérer à l'œil nu, en particulier par un chirurgien expérimenté. La zone de contact Z3 est à nouveau un unique point R3 disposé sur le processus épineux 5 dans une zone tangentielle. En conséquence, les parties de contact convexes 21, 22 sont chacune destinées à venir en contact avec les zones de références concaves Z1, Z2 respectivement et la troisième partie de contact 23 est posée sur le point R3 afin de déterminer un système de coordonnées ou « référentiel d'orientation » RO de la zone opératoire ZO par rapport au référentiel d'orientation RS de la salle opératoire ZS.

L'outil chirurgical 30 est par exemple un outil pour percer la vertèbre 1, et comprend : une tige 31 ; un point 32 à l'extrémité devant de la tige ; une poignée 33 ; un moyen de détermination 34 d'un référentiel d'orientation RT de l'outil ; et un moyen de communication du référentiel d'orientation RT de l'outil.

Dans le cas le plus simple, les moyens de détermination 24, 34 sont des niveaux à bulle, aussi parfois appelés inclinomètre, et les moyens de communication 25, 36 sont des indicateurs visuels, par exemple des chiffres marqués autour du niveau à bulle ou même un simple cercle au centre du niveau.

En variante, les moyens de détermination 24, 34 peuvent être des systèmes « MEMS » ou « microsystème électromécanique » qui déterminent le plan de l'ancillaire 20. Ces moyens peuvent comprendre un accéléromètre tri-axe, un gyroscope tri-axe, et/ou un magnétomètre tri-axe, comme connu par l'homme du métier et par conséquent non expliqué plus en détails. Les moyens de communication 45 peuvent être une liaison filaire (câble) ou non-filaire (sans contact), par exemple par Wi-Fi ou Bluetooth.

Dans les modes de réalisation illustrés sur la figure 2A, les deux éléments concaves/convexes 21, 22 sont de forme sensiblement régulière autour d'un axe, et leurs axes générateurs sont coaxiaux (selon l'axe Δ).

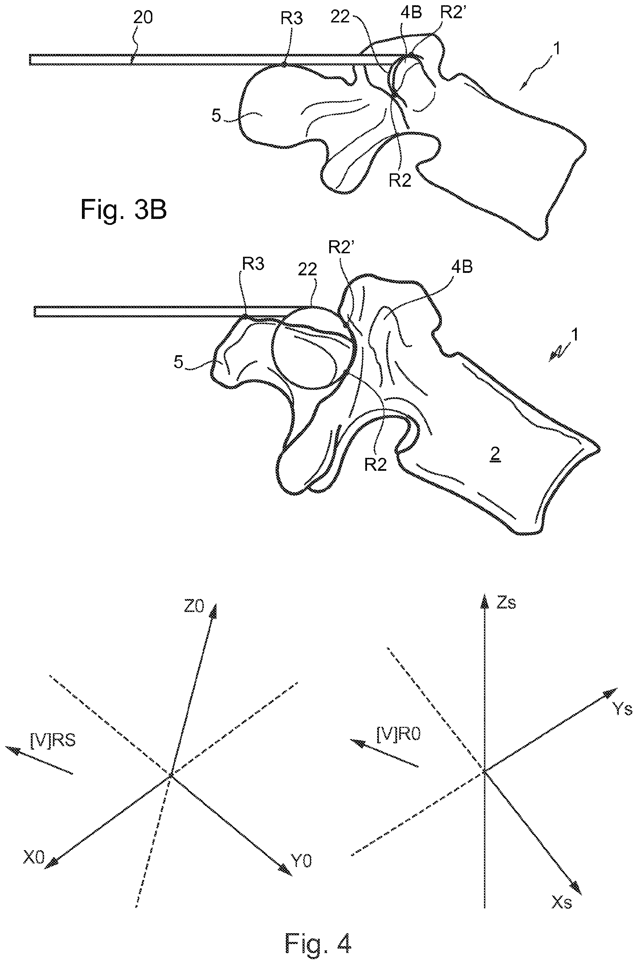

Les parties de gauche des **figures 3A, 3B** représentent respectivement une vue de dessus et une vue de côté de l'ancillaire 20 du SYS1 posé sur une vertèbre thoracique 1. Les parties de droite des **figures 3A, 3B** représentent respectivement une vue de dessus et une vue de côté de l'ancillaire 20 du SYS2 posé sur une vertèbre lombaire 1.

Les dimensions de l'ancillaire 20 sont adaptées à l'application envisagée, par exemple environ 10 cm de largeur, 15 cm de longueur, et 0.50 cm d'épaisseur pour les opérations sur la colonne vertébrale humaine, avec un diamètre des gouttières d'environ 1 cm.

Dans une phase préopératoire, des images sont obtenues, par exemple de toute la colonne vertébrale, pour faire des reconstructions en trois-dimensions de la zone opératoire. Ensuite, pour chaque vertèbre, on détermine les zones de référence Z1, Z2, Z3, pour définir le référentiel d'orientation RO de la zone opératoire. Il peut s'agir, pour un opérateur, d'indiquer des zones d'intérêt. Puis, on détermine par exemple deux points de référence R1, R1', R2, R2' pour chacune des zones Z1, Z2. Ces points sont aisément déterminables par un ordinateur compte tenu de la représentation 3D de la vertèbre considérée et de l'ancillaire utilisé. Il s'agit par exemple de points les plus saillants ou proéminents (vers l'extérieur ou vers l'intérieur) qui viendront en contact avec les éléments 21, 22 de l'ancillaire. En d'autres termes, il s'agit de points de contact compte tenu des dimensions de l'ancillaire retenu (diamètre de la partie convexe/concave, etc.).

Dans certains modes de réalisation, le point R3 est un point dans la zone tangentielle Z3, qui sera plus difficile à déterminer à l'œil nu mais facilement déterminable par un programme d'ordinateur, qui rentrera en contact avec la troisième partie de contact 23 par simple pose de l'ancillaire 20 sur la zone Z3.

La **figure 3B** montre que la partie de contact concave 22 rentre en contact avec uniquement deux points R2, R2' de la zone de référence Z2 formée du processus transversal 4B (bien que la figure montre des points de contact dans le même plan transversal, ceux-ci peuvent être décalés longitudinalement). Il en est de même pour la partie de contact concave 21 (non représentée). Le choix de parties de contact concaves 21, 22 présentant un rayon de courbure supérieur au rayon de courbure local des zones de référence convexes Z1, Z2 correspondantes garantit, grâce aux irrégularités anatomiques de la zone de référence, que le contact se fait uniquement au niveau de deux points. Le rayon de courbure local peut correspondre au rayon d'une approximation de la zone de référence, c'est-à-dire substantiellement la zone où un ou deux contacts vont avoir lieu. Le rayon de la partie concave et le rayon de courbure local s'apprécient selon une même section transversale.

De façon similaire, la partie de contact convexe 22 rentre en contact avec uniquement deux points R2, R2' de la zone de référence Z2 formée de la lame 6B (de même pour la partie de contact convexe 21 non représentée). Le choix de parties de contact convexes 21, 22 présentant un rayon de courbure supérieur au rayon de courbure des zones de référence concaves Z1, Z2 correspondantes garantit que le contact se fait uniquement au niveau de deux points.

On voit de ces figures que le basculement en rotation de l'ancillaire 20 est facilité par un guidage de celui-ci par les points de référence R1, R1', R2, R2', ceux-ci permettant de définir l'axe de rotation Δ vis à vis de l'os.

Ensuite, dans le cas du placement des vis pédiculaires, la saisie des directions de vissage optimal des vis pédiculaires 12 permet de définir un vecteur directeur pour chaque vis pédiculaire.

En référence à la **figure 4,** qui représente le référentiel d'orientation RO de la zone opératoire et le référentiel d'orientation galiléen RS de la salle opératoire, chaque référentiel d'orientation RO, RS comprend trois axes, Xo, Yo, Zo ; Xs, Ys, Zs respectivement. Une flèche [V]ro (ou « référentiel local ») représente un vecteur directeur V d'un geste chirurgical (par exemple la pose d'une vis pédiculaire) exprimée dans le référentiel d'orientation RO de la zone opératoire (actuellement dans la vertèbre). Le référentiel d'orientation RO de la zone opératoire n'est pas nécessairement aligné avec le référentiel RS de la salle d'opération, comme montré à la **figure 4.**

Ensuite, pendant la phase peropératoire, le chirurgien positionne l'ancillaire 20 sur la vertèbre 1, comme montré aux **figures 3A, 3B,** en mettant les parties de contact 21, 22 en contact avec les zones de références Z1, Z2, c'est-à-dire jusqu'à obtenir un contact avec les points de référence R1, R1' et R2, R2' respectivement, et ensuite en basculant, par rotation, l'ancillaire 20 alors que les parties de contact 21, 22 sont en contact constant avec les zones de référence respectivement, de sorte à poser la partie de contact 23 sur la zone de référence Z3. On note que le basculement s'effectue par rotation autour de l'axe des parties de contact 21, 22, c'est-à-dire autour de l'axe de révolution des portions courbes formant ces parties de contact (l'axe Δ).

Le référentiel d'orientation RO de la zone opératoire ZO est alors déterminé par rapport au référentiel RS de la salle opératoire, grâce aux moyens de détermination et de communication 24, 25 du référentiel d'orientation RO de la zone opératoire ZO.

Une matrice de rotation Mrors, qui exprime le référentiel d'orientation RO dans le référentiel d'orientation RS, est définie. Un vecteur directeur [V]rs dans le référentiel d'orientation RS de la salle opératoire ZS peut être établi par rapport au référentiel d'orientation RO de la zone opératoire ZO, précédemment établi, selon l'équation suivante :

$$[V]rs = Mrors \cdot [V]ro \qquad\qquad [\text{équation 1}]$$

Enfin, l'outil chirurgical 30 détermine et communique, grâce aux moyens de détermination et de communication 34, 35 du référentiel d'orientation RT de l'outil, en temps réel son orientation, notamment l'orientation de sa tige 31, dans le référentiel d'orientation RS de la salle opératoire. L'orientation dynamique de la tige 31 par rapport à l'orientation idéale de la vis pédiculaire à poser, permet au chirurgien d'adapter l'orientation de l'outil 30 pour la faire correspondre à l'orientation du vecteur directeur [V]ro exprimé dans le référentiel galiléen RS (soit [V]rs).

En variante, le vecteur directeur [V]ro et l'orientation de l'outil 30 peuvent être convertis dans un référentiel tiers, par exemple un référentiel utilisé pour un affichage, pour permettre au chirurgien de visualiser lorsque l'orientation de l'outil 30 correspond à l'orientation du geste chirurgical, dans ce référentiel.

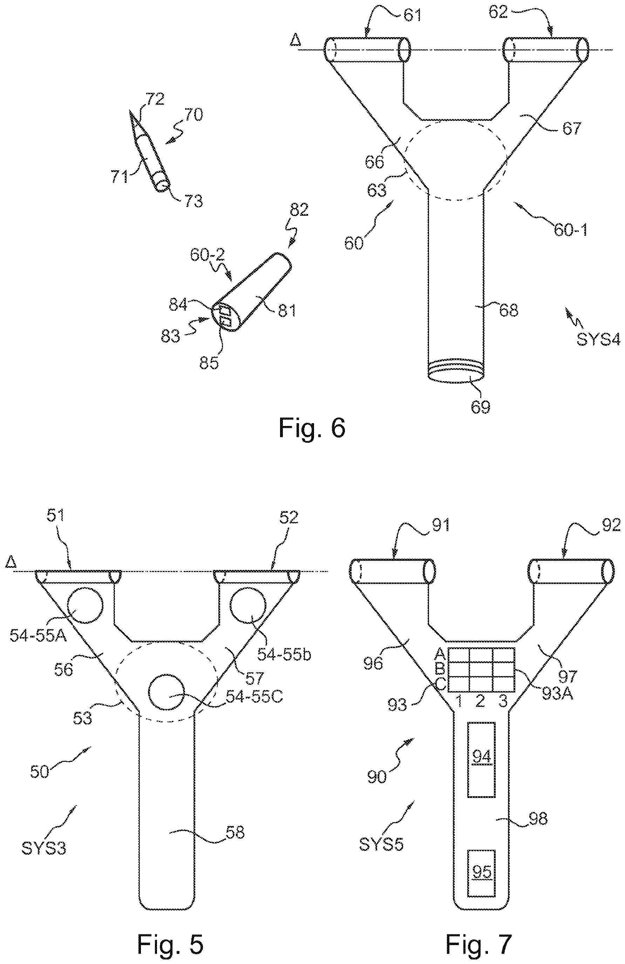

La **figure 5** représente une vue de dessus d'un système d'orientation chirurgical SYS3 selon un autre mode de réalisation. Le système SYS3 comprend un ancillaire 50 et un outil chirurgical (non montré pour des raisons de simplicité).

L'ancillaire 50 comprend : au moins deux parties de contact 51, 52, concaves et/ou convexes comme décrites précédemment ; une troisième partie de contact 53, par exemple tangentiel ; et des moyens de détermination et de communication 54-55A, 54-55B, 54-55C du référentiel d'orientation RA (RO). Les moyens 54-55A, 54-55B, 54-55C sont des marqueurs optiques non-alignés et destinés à être captés par une pluralité de caméras qui filment la salle d'opération ZS en temps réel, afin de repérer les positions des outils par rapport au modèle.

Similairement à l'ancillaire 20 décrit en relation avec la **figure 2A,** dans ce mode de réalisation, l'ancillaire 50 est en forme de Y (« i-grec »), les parties de contact 51, 52 étant des éléments concaves et/ou convexes (par exemple l'un en forme de gouttière et l'autre en forme de cylindre) d'une première branche 56 et d'une deuxième branche 57 respectivement, et une troisième branche 58 servant de poignée. Les branches 56, 57 sont les extrémités supérieures gauche et droite de l'Y respectivement, la branche 58 est l'extrémité inférieure centrale de l'Y et la partie de contact 53 est disposée au centre de l'Y.

L'outil chirurgical peut être similaire à l'outil 30 décrit en relation avec la **figure 2A** (comprenant des inclinomètres), comporter des moyens de détermination MEMS et de communication filaire ou non-filaire (sans contact) ou même comporter des marqueurs optiques.

En outre, il n'est pas obligatoire que l'outil chirurgical comporte des tels moyens de détermination et de communication. Dans ce cas, il peut être un simple outil chirurgical classique.

La **figure 6** représente une vue de perspective d'un système d'orientation chirurgical SYS4 selon un autre mode de réalisation. Le système SYS4 comprend un ancillaire 60 et un outil chirurgical 70. Dans ce mode de réalisation, l'ancillaire 60 est divisé en deux parties, une première partie 60-1 pour la prise de contact avec la zone opératoire, et une deuxième partie 60-2 de détermination et de communication du référentiel d'orientation de la salle opératoire.

La première partie 60-1 de l'ancillaire 60 est similaire à l'un quelconque des ancillaires 20, 50 décrit précédemment, et comprend : au moins deux parties de contact 61, 62 ; une partie de contact tangentiel 63 ; des branches 66, 67, 68 ; et une extrémité 69 pour recevoir la deuxième partie 60-2.

La deuxième partie 60-2 comprend : un corps 81 ; une extrémité devant 82 creuse pour recevoir l'extrémité derrière 69 de la première partie 60-1; un moyen de détermination 84 du référentiel d'orientation RA, RT (RO) de la partie 60-1 de l'ancillaire 60 ; et un moyen de communication 85 du référentiel d'orientation de l'ancillaire 60.

L'outil chirurgical 70 comprend : une tige 71 ; un point 72 à l'extrémité devant de la tige ; et une extrémité derrière 73.

De préférence, l'ancillaire 60 et l'outil chirurgical 70 coopèrent afin que la deuxième partie 60-2 de l'ancillaire 60 puisse être encastrée sur la première partie 60-1 et l'outil 70 d'une manière non définitive (elle peut être enlevée), précis (pas de jeu entre les éléments) et répétable (reproductible). A cette fin, les extrémités derrières 69, 73 de la première partie 60-1 de l'ancillaire 60 et de l'outil chirurgical 70 respectivement peuvent comprendre des saillies reçus dans une encoche à l'intérieur de l'extrémité devant 82 creuse, obligeant la deuxième partie 60-2 d'être encastré d'une manière préalablement défini.

La deuxième partie 60-2 est d'abord encastrée sur l'extrémité 69 de la première partie 60-1 de l'ancillaire 60. Une fois le référentiel d'orientation RA déterminé et communiqué, la partie 60-1 est mise de côté et la partie 60-2 est enlevée et posée sur l'extrémité de l'outil chirurgical 70 pour déterminer et communiquer à nouveau le référentiel d'orientation RT de l'outil 70. Ce système permet une réduction de coût car un seul dispositif de détermination et de communication de référentiel est nécessaire, et peut être utilisé dans le cas où la zone opératoire n'est pas susceptible de changer de position en cours d'opération.

La **figure 7** représente une vue de dessus d'un système d'orientation chirurgical SYS5 selon un autre mode de réalisation. Le système SYS5 comprend un ancillaire 90 et un outil chirurgical (non montré pour des raisons de simplicité).

L'ancillaire 90 comprend au moins deux parties de contact 91, 92 ; une troisième partie de contact 93, par exemple tangentiel ; des moyens de détermination 94 du référentiel d'orientation, par exemple un système « MEMS », et des moyens de communication 95 du référentiel d'orientation par exemple une liaison non-filaire (sans contact).

Similairement à l'ancillaire 50 décrit en relation avec la **figure 5,** dans ce mode de réalisation, l'ancillaire 90 est en forme de Y (« i-grec ») avec les parties de contact concaves et/ou convexes (des cylindres sur la figure) 91, 92 disposées à l'extrémité d'une première branche 96 et d'une deuxième branche 97 respectivement, et avec une troisième branche 98 servant de poignée. Les branches 96, 97 sont les extrémités supérieures gauche et droite de l'Y respectivement, la branche 98 est l'extrémité inférieure centrale de l'Y et la partie de contact 93 est disposée au centre de l'Y.

L'ancillaire 90 comprend en outre des moyens de validation 93A de la partie de contact tangentiel 93. A cette fin, dans ce mode de réalisation, la partie de contact 93 est transparente et agencée d'une forme quadrillée et de préférence marquée par exemple par des chiffres (1 à 3) et des lettres (A à C), afin de permettre à un chirurgien de déterminer où exactement la zone tangentielle est en contact avec un point de référence R3 de la troisième zone de référence Z3. Par exemple, pendant la phase préopératoire, il peut être déterminé que le point de référence R3 doit être en contact avec la zone A3 de la zone tangentielle. Les parties de contact 91, 92 peuvent être aussi équipées de marqueurs (non-montrés) afin de faciliter le repérage des points de contact R1, R1', R2, R2'.

Dans une autre mode de réalisation, les moyens de validation sont une zone de contact sensible qui détecte le contact avec le troisième point de référence, et le communique par exemple par les moyens de communication.

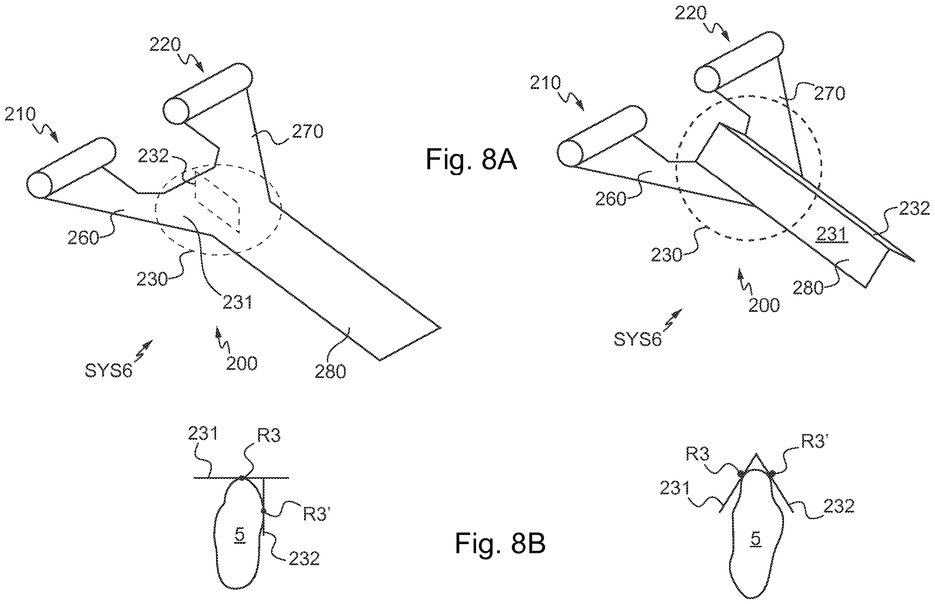

La **figure 8A** représente des vues en perspective d'un système d'orientation chirurgical SYS6 selon un autre mode de réalisation. Le système SYS6 comprend un ancillaire 200 et un outil chirurgical (non montré pour des raisons de simplicité).

L'ancillaire 200 est en forme de Y et comprend deux premières parties de contact concaves et/ou convexes (des cylindres sur la figure) 210, 220 disposées aux extrémités d'une première branche 260 et d'une deuxième branche 270 respectivement. Les deux parties de contact 210, 220 peuvent être de n'importe quel type précédemment évoqué. Les éventuels moyens de détermination d'un référentiel d'orientation et de communication ne sont pas représentés.

L'ancillaire 200 se singularise par la géométrie de la troisième partie de contact 230, qui n'est plus une simple surface plane réalisant un contact tangentiel avec la zone opératoire. La troisième partie de contact 230 est formée d'une zone concave (comme sur la figure) ou convexe (non représentée) destiné à venir en contact avec deux points de référence R3, R3' de la troisième zone de référence Z3 qui présente une forme inverse, c'est-à-dire convexe ou concave respectivement.

Dans l'exemple de la **figure 1A,** la surface supérieure du processus épineux 5 présente une forme convexe. Une troisième partie de contact concave 230 est donc préférentiellement utilisée.

Comme montré sur la **figure 8A,** la troisième partie de contact 230 peut être formée de deux surfaces planes 231, 232, préférentiellement disposées en V inversé ou T, et destinées chacune à venir en contact avec un unique point de référence R3 ou R3' de la troisième zone de référence Z3.

Dans le premier exemple de la **figure 8A,** la première surface place 231 est constituée du corps central de l'ancillaire en Y, signifiant que les première et deuxième parties de contact 210, 220 (et donc les branches 260, 270) et cette surface plane 231 font partie d'une même pièce monobloc. La deuxième surface 232 est alors constituée d'une pièce mécanique rapportée (de préférence de façon solidaire, éventuellement amovible) sur la pièce monobloc formant Y.

Dans l'autre exemple, la troisième branche 280 formant manche n'est pas plane mais faite de deux plaques planes 231, 232 formant, en section, un V inversé. Ainsi, la troisième zone est de type gouttière ayant un profil en section en forme de V inversé.

La **figure 8B** représente une vue en coupe de l'ancillaire 200 en position dans les exemples des **figures 3A** et **3B,** au niveau du processus épineux 5 où se trouve la troisième zone de référence Z3. La présence des deux points de contact R3, R3' (représentés ici dans la même coupe en section pour des raisons de clarté, alors qu'en pratique ils peuvent être décalés selon l'axe longitudinal du processus épineux) garantit un positionnement exact, et donc reproductible, de l'ancillaire 200 sur la zone opératoire, ici une vertèbre.

La zone concave ou convexe formant troisième partie de contact 230 n'est pas nécessairement la réunion de deux surfaces planes comme sur les **figures 8A** et **8B.** Il peut s'agir par exemple d'un agencement (gouttière, cylindre, etc.) présentant une paroi de section partiellement ou entièrement circulaire ou de section parabolique.

Ces exemples d'ancillaires ne sont que des modes de réalisation de l'invention qui ne s'y limite pas. Par exemple, les modes de réalisation illustrés ci-dessus présentent principalement deux extrémités 21, 22 (ou 51, 52 ; 61, 62 ; 91, 92 ; 210, 220) de section courbe ayant des axes générateurs (Δ) sensiblement coaxiaux.

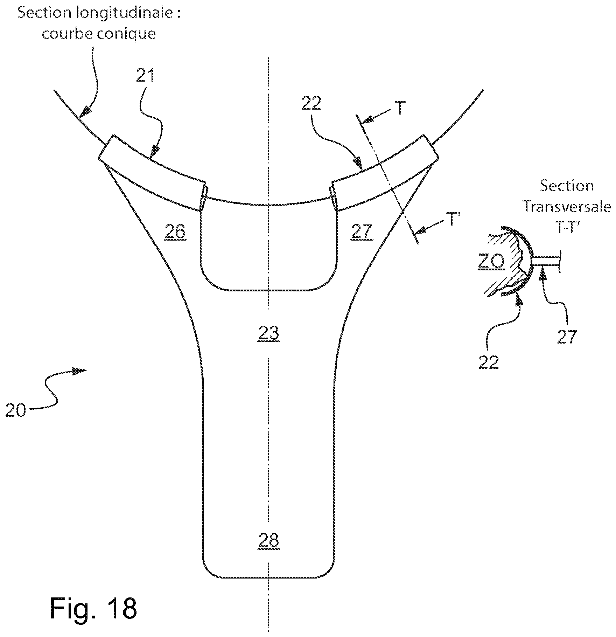

Dans une variante illustrée en **Figure 18,** les deux extrémités présentent toujours des sections transversales courbes (convexes ou concaves, selon la coupe T-T'), mais présentent également des sections longitudinales (section selon le plan de l'ancillaire) qui appartiennent à une même courbe conique, par exemple à un cercle d'axe perpendiculaire formé par le plan de l'ancillaire en Y, une ellipse dans ce même plan, ou encore une parabole. Cela permet de faciliter la rotation de l'ancillaire dans un plan contenant l'ancillaire. Cette facilité de rotation peut par exemple venir compenser un défaut de basculement/pivotement à raison de contacts indésirables avec la zone opératoire ZO. Cette facilité de rotation peut aussi être utilisée pour atteindre la position dans laquelle les deux surfaces planes 231, 232 de la troisième partie de contact 230 (voir les réalisations des **Figures 8)** sont mises en contact avec la zone opératoire aux points R3 et R3'.

La **figure 9** représente une salle opératoire ZS équipée d'un système d'orientation chirurgical. A titre d'exemple, un système SYS1' est montré ici, comprenant un ancillaire 20' et un outil 30' équipés par des moyens de détermination « MEMS » et de moyens de communication non-filaire.

La salle opératoire ZS est équipée d'un dispositif 110 d'affichage d'images et de traitement de données, tel un ordinateur. Le dispositif 110 comprend un écran 111, un processeur 112, des moyens d'entrée et de manipulation de données 113 (un clavier, un souris, un capteur de voix, une surface tactile, etc.), et des moyens de réception 114 des données communiquées par l'ancillaire 20' et/ou l'outil 30'.

La salle opératoire comprend en outre un « entité opératoire » 100 comprenant un chirurgien 101 qui opère sur un patient 102 allongé sur une table d'opération 103.

L'écran 111 permet d'afficher des images I obtenues de la zone opératoire ZO pendant la phase préopératoire. Le personnel du bloc opératoire, et particulièrement le chirurgien, peut consulter les images pendant l'opération. Ces images peuvent être « statiques » ou avantageusement « dynamiques ». Par dynamique, il est entendu que les référentiels de l'ancillaire 20' et/ou de l'outil chirurgical 30' sont déterminés, communiqués à l'ordinateur 110, et affichés sur l'écran 111 en temps réel. Le chirurgien 101 peut alors avoir une idée précise de l'orientation de ses outils par rapport à la vertèbre.

Dans un mode de réalisation, le système est interactif et permet au chirurgien 101 de donner des instructions orales, par exemple « Affichez la vertèbre L5. » afin que l'ordinateur affiche l'image correspondant à la vertèbre L5.

La **figure 10** représente un organigramme d'une phase préopératoire P1, et la **figure 11** représente un organigramme d'une phase peropératoire P2.

La phase P1 comprend les étapes S1 à S5. A l'étape S1, au moins une image I d'au moins une zone opératoire ZO est prise, par exemple par un moyen de tomodensitométrie. A l'étape S2, au moins trois zones de référence Z1, Z2, Z3 sont déterminées ou indiquées par un opérateur et enregistrées dans le cas d'un système dynamique, ou simplement notées dans le cas d'un système statique. Au moins une des zones Z1 et Z2 est définie à l'aide de deux points de référence R1, R1' ou R2, R2' pour lesquels on cherchera un contact avec l'ancillaire 20'.

Dans le cas des **figures 2** à **7**, deux paires de deux points de référence (R1, R1') et (R2, R2') sont déterminées, et un unique point de référence R3 est déterminé pour la zone de référence Z3 située sur le processus épineux 5 dans ces exemples.

Dans le cas des **figures 8A** et **8B**, trois paires de deux points de référence (R1, R1'), (R2, R2') et (R3, R3') sont déterminées.

Ces multiples points de référence sont déterminés par l'ordinateur 110, notamment comme étant des points saillants ou proéminents sur les zones de référence indiquées, sur lesquels l'ancillaire 20' viendra en contact. Par exemple, compte tenu des dimensions géométriques d'un ancillaire choisi, l'ordinateur 110 est capable de déterminer lesquels parmi un ensemble de points saillants viendront en contact avec l'ancillaire. L'ancillaire est notamment choisi pour satisfaire les conditions de rayon de courbure évoquées précédemment, étant entendu que le rayon de courbure des zones de références visées peut être estimé par modélisation et approximation de la surface (concave ou convexe) de ces zones de références.

A l'étape S3, un référentiel opératoire RO, comme décrit plus haut en relation avec la **figure 4**, est calculé au moyen des points de référence et ensuite enregistré ou noté. A l'étape S4, au moins un vecteur directeur [V]ro est déterminé pour le geste chirurgical à réaliser et ensuite enregistré ou noté. A l'étape S5, le procédé est répété si nécessaire pour d'autres zones opératoires.

La phase P2 comprend les étapes S11 à S16. A l'étape S11, la zone opératoire ZO est exposée. A l'étape S12, une partie de contact 21' de l'ancillaire 20' est mise en contact avec la zone de référence correspondante Z1, par exemple en contact avec deux points de référence R1, R1'. Il peut s'agir d'engager la partie de contact convexe dans la zone de référence concave correspondante (comme dans la partie de droite de la **figure 3B** avec un cylindre), ou d'engager la partie de contact concave sur la zone de référence convexe correspondante (c'est-à-dire d'envelopper la zone de référence convexe comme montré dans la partie de gauche de la **figure 3B** avec une gouttière).

A l'étape S13, l'autre partie de contact 22' de l'ancillaire 20' est mise en contact avec la deuxième zone de référence Z2, de façon identique à la première partie de contact si elles sont toutes deux concaves et/ou convexes.

A ce stade, l'ancillaire 20' est engagé, par les deux parties de contact 21', 22', sur la zone opératoire. Les trois ou quatre points de contact R1, R1', R2, R2' peuvent ainsi servir de guidage en rotation de l'ancillaire. Avantageusement, ces quatre points de contacts sont tangentés par le cylindre concave ou convexe (dépendamment de l'ancillaire utilisé) qui détermine ainsi un axe de rotation.

A l'étape S14, l'ancillaire 20' est basculé en rotation, en conservant les contacts R1, R1', R2, R2' sans un grand effort de l'opérateur. La troisième partie de contact 230 de l'ancillaire 20' est alors mise en contact avec la troisième zone de référence Z3, par exemple simplement posée sur le point de référence R3 (exemples des **figures 2** à **7**), ou mis en contact avec les deux points de référence R3, R3' (exemples des **figures 8A** et **8B**).

A l'étape S15, le référentiel d'orientation RO de la zone opératoire ZO est déterminé et communiqué, permettant le calcul de la matrice de rotation Mrors et du vecteur directeur [V]rs à l'aide de l'équation 1. Ainsi, l'ancillaire selon l'invention permet de déterminer simplement et rapidement l'orientation de la zone opératoire, par exemple une vertèbre, aux fins de réaliser un acte, par exemple un acte chirurgical.

A l'étape S16, l'outil chirurgical est utilisé pour réaliser un geste chirurgical selon le vecteur directeur calculé.

La **figure 12** représente un support non transitoire 120 lisible par un ordinateur et comprenant une programme d'instructions 121 exécutable par ordinateur. Le programme d'instructions peut comprendre l'algorithme de calcul décrit en relation avec la **figure 4**.

Des modes de réalisation concernent en outre un ensemble ou « kit » d'au moins deux ancillaires 20, 20', 50, 60, 90, 200, chaque ancillaire étant conçue pour des zones opératoires ZO différentes l'une l'autre, par exemple ayant des dimensions différentes, des angles différents entre les branches, des premières et deuxièmes parties de contact différentes, des troisièmes parties de contact différentes, etc. Ceci permet de couvrir une gamme de variations anatomiques. Dans un mode de réalisation, les ancillaires 20, 20', 50, 60, 90, 200 ont différent tailles, par exemple petit, moyen, et large.

Il sera compris par l'homme du métier que les modes de réalisation décrits en ce qui précède peuvent être modifiés.

Par exemple, les moyens de communication 25, 35, 55, 65 peuvent être un écran numérique, des diodes électroluminescentes DEL (« LED » ou « Light-Emitting Diode » en anglais) par exemple vertes, oranges et rouges qui s'allument, des liaisons filaires (un câble connecté à l'outil chirurgical ou au dispositif de traitement de données), non-filaires (Wi-Fi, NFC, Bluetooth, etc), un signal auditif et, plus généralement, tout moyen de communication d'information.

En ce qui précède, les zones de contact 23, 53, 63, 93 ont été décrites comme des zones essentiellement planes qui se posent sur une zone de contact tangentiel ZT. (Par « essentiellement planes » il est compris que la zone est plus ou moins planaire dans les limites de fabrication). Néanmoins, il sera compris par l'homme du métier que ces zones de contact peuvent avoir toute d'autre forme conçue pour venir en contact avec une zone déterminée.

Dans un mode de réalisation, non montré, un outil de détermination et de communication d'un référentiel d'orientation est fixé sur la zone opératoire ZO elle-même afin de vérifier en permanence sa position, par exemple pour s'assurer que le patient n'a pas bougé pendant l'opération, pour les opérations très délicates.

La position du patient, et plus particulièrement de la zone opératoire, peut être ajustée jusqu'à ce que la bonne orientation soit trouvée. Des moyens (des sangles, des pinces, etc.) pour tenir la zone opératoire (le patient) dans une position donnée peuvent être mis en oeuvre, soit avant l'opération, soit pendant l'opération.

Il sera compris par l'homme du métier que certains éléments décrits en relation avec un mode de réalisation (par exemple les moyens de détermination et de communication, les « MEMS », etc.) peuvent être appliqués aux autres modes de réalisation.

Notamment une partie de contact convexe peut être combinée à une partie de contact concave (voir par exemple la **figure 5**) au sein du même ancillaire. En outre, une première partie de contact (concave ou convexe) peut être combinée à une deuxième partie de contact qui peut réaliser un contact avec la zone opératoire en un unique point. Par exemple, la deuxième partie de contact peut être formée d'une arête rectiligne (munie ou non de picots antidérapants) ou d'un pointeau destiné à venir en contact avec un unique point de référence de la deuxième zone de référence Z2.

Par ailleurs, une partie convexe peut être de tout type : rouleau, galet, cylindre, sphère, demi-cylindre ou cylindre partiel (la section est un cercle partiel), ou plus généralement une forme arrondie. Une partie concave peut être de tout type : gouttière de section partiellement circulaire ou parabolique ou en V, coupelle sphérique, paroi d'extrémité semi-cylindrique ou partiellement cylindrique.

Ces parties convexes/concaves terminales des branches de l'ancillaire en Y peuvent être solidaires des branches, ou être montées mobiles en rotation.

Les axes de révolution des parties convexes/concaves (les axes de rotation le cas échéant) peuvent être coaxiaux ou décalés voire inclinés l'un par rapport à l'autre (c'est-à-dire non parallèles), selon l'anatomie de la zone opératoire.

L'outil chirurgical est par exemple un perforateur, un tournevis et, en général, tout outil qui permet de réaliser un acte chirurgical.

Les matériaux utilisés pour l'ancillaire et l'outil chirurgical peuvent, de préférence, être stérilisés et ne posent pas de problème de biocompatibilité.

Comme évoqué plus haut, dans certains modes de réalisation, il n'est pas obligatoire que l'outil chirurgical soit équipé d'un moyen de détermination et de communication de l'orientation. Dans certains cas, une fois que le référentiel d'orientation RO a été obtenu, le chirurgien peut facilement déterminer lui-même l'angle correct, par exemple un angle de 90° par rapport au référentiel d'orientation de l'ancillaire.

Dans certains modes de réalisation, les branches de l'ancillaire 20, 20', 50, 60, 90, 200 peuvent être articulées autour de la zone centrale, par exemple aux moyens des charnières disposés entre la zone centrale et chaque branche.

Enfin, d'autres modes de calcul des vecteurs directeurs peuvent être mises en oeuvre.

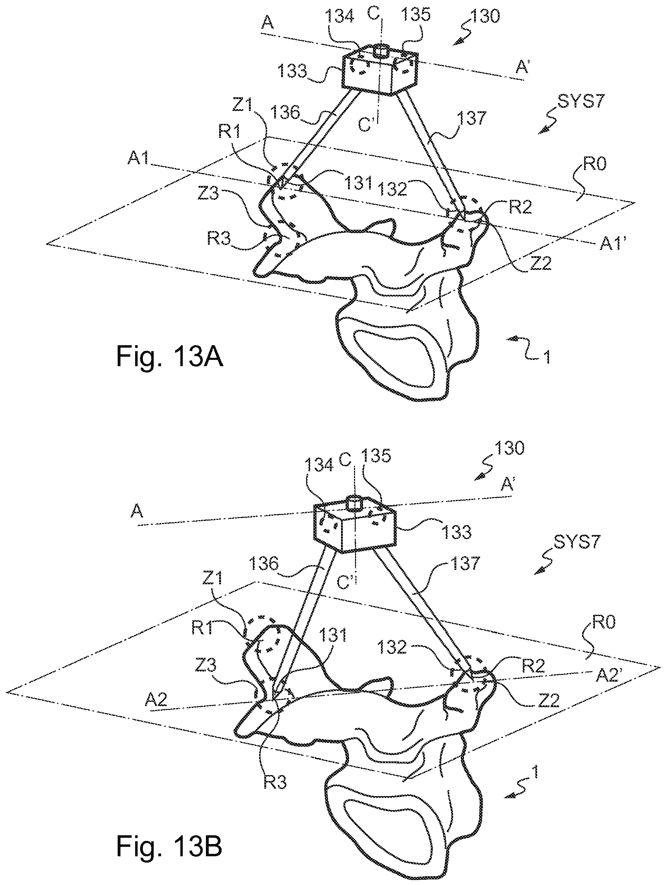

Les **figures 13A** et **13B** représentent des vues en perspective d'un système d'orientation simplifié SYS7 selon un exemple ne faisant pas partie de l'invention, dans deux positions différentes de mesure.

Le système SYS7 comprend un ancillaire 130 et un outil (non montré pour des raisons de simplicité). L'ancillaire 130 est en forme de compas à deux branches. Il comprend : une première branche 136 ayant une première extrémité terminale de contact 131 destinée à venir en contact avec une première zone de référence Z1 d'une zone d'intervention 1 ; une deuxième branche 137 ayant une deuxième extrémité terminale de contact 132 destinée à venir en contact avec une deuxième zone de référence Z2 de la zone d'intervention 1 ; une partie fixe ou « carter » 133 relativement à laquelle les deux branches 136, 137 se déplacent pour ouvrir ou fermer l'ancillaire 130, de sorte que, lors de l'ouverture ou de la fermeture de l'ancillaire, les première et deuxième extrémités terminales 131, 132 forment un axe (A1-A1') constamment parallèle à un axe de référence (A-A') de la partie fixe 133. Le carter 133 peut servir de moyen de préhension (ou poignée) pour qu'un opérateur puisse manipuler l'ancillaire.

L'ancillaire 130 comprend en outre un moyen de détermination 134 d'une orientation de l'ancillaire dans un référentiel galiléen et, optionnellement, un moyen de communication 135 des orientations ainsi déterminées par le moyen 134 (non représentés en détail). Comme expliqué par la suite, le moyen de détermination d'une orientation 134 peut être inclus dans un moyen de détermination d'un référentiel d'orientation ou d'intervention RA (et en conséquence RO) dans un référentiel galiléen. Dans ce cas, le moyen de communication 135 est plus apte à communiquer le référentiel d'orientation ainsi déterminé.

Ces moyens 134, 135 peuvent éventuellement être prévus amovibles comme dans la **figure 6.**

Dans cet exemple, le moyen de détermination 134 est un système « MEMS » ou « microsystème électromécanique », de type centrale inertielle, qui détermine l'orientation de l'axe (A1-A1') dans le référentiel galiléen. Ce moyen peut comprendre un accéléromètre tri-axe, un gyroscope tri-axe, et/ou un magnétomètre tri-axe, comme connu par l'homme du métier et ne sera pas expliqué plus en détail. Le moyen de communication 135 est une liaison filaire (câble) ou non-filaire (sans contact), par exemple par Wi-Fi ou Bluetooth, pour transmettre cette orientation à un système de calcul distant.

Dans l'exemple illustré ici, les première et deuxième branches 136, 137 de dimensions sensiblement égales sont montées mobiles en rotation sur la partie fixe 133 et sont agencées pour s'ouvrir ou se fermer de façon symétrique relativement à la partie fixe 133. Ainsi, l'écartement des branches 136, 137 de l'ancillaire peut être ajusté en fonction de l'écartement entre les zones de référence Z1, Z2, pour s'adapter, avec un ancillaire unique, aux dimensions fortement variables de plusieurs zones d'intervention.

Toujours dans cet exemple, la centrale inertielle 134 et les éventuels moyens de transmission 135 sont montés solidaires de la partie fixe 133. Ainsi, l'orientation (A1-A1') à déterminer est la même que l'orientation (A-A') de la centrale inertielle 134 (et du carter 133). Un unique moyen de détermination 134 (par exemple une unique centrale inertielle) est donc suffisant.

Sur la figure, trois zones de références Z1, Z2, Z3 sont définies qui comprennent chacune un seul point de référence. Les trois points R1, R2, R3 sont non alignés. Les extrémités terminales 131, 132 des branches 136, 137 de l'ancillaire 130 sont formées de pointes destinées à venir en contact avec chacun des points de référence R1, R2, R3.

Bien entendu, dans une variante, l'une ou l'autre des deux extrémités 131, 132 (voire les deux) peut être formée d'une extrémité concave ou convexe destinée à venir en contact avec au moins deux points de référence de la première ou deuxième zone de référence correspondante qui est de forme convexe ou concave respectivement. Il peut s'agir de l'une quelconque des configurations exposées ci-dessus en lien avec les **figures 1 à 12.**

L'ancillaire 130 peut être utilisé de façon simple pour déterminer *in situ* un référentiel d'intervention d'une zone d'intervention, dans un référentiel d'orientation galiléen.

Pour ce faire, deux mesures sont effectuées comment montrées sur les **figures 13A** et **13B,** en positionnant successivement les deux extrémités terminales 131, 132 de l'ancillaire sur chacune de deux paires de zones de référence (Z1 et Z2 sur la **figure 13A,** et Z2 et Z3 sur la **figure 13B).** Le moyen de détermination 134 obtient ainsi au moins deux orientations non parallèles dans le référentiel d'orientation galiléen : (A1-A1') lors de la mesure en **figure 13A** et (A2-A2') lors de la mesure en **figure 13B.**

Ces deux orientations permettent, à elles-seules, de déterminer le référentiel d'orientation ou d'intervention de la zone d'intervention 1, dans le référentiel galiléen.

Le premier axe mesuré (A1-A1') peut être utilisé comme premier axe du référentiel d'intervention, et on peut lui associer un vecteur unitaire. Le produit vectoriel de ce vecteur unitaire avec un vecteur porté par le deuxième axe mesuré (A2-A2') permet alors de définir un deuxième axe du référentiel d'intervention. On peut également lui associer un vecteur unitaire. Le produit vectoriel des deux vecteurs unitaires permet de définir le troisième axe du référentiel d'intervention, dans le référentiel d'orientation galiléen. On lui associe également un vecteur unitaire. Ainsi, un référentiel orthonormé est obtenu.

Ces opérations peuvent être réalisées dans le moyen de détermination 134 ou dans un système de calcul distant auquel les orientations (A1-A1') et (A2-A2') auront été transmises par le moyen de communication 135.

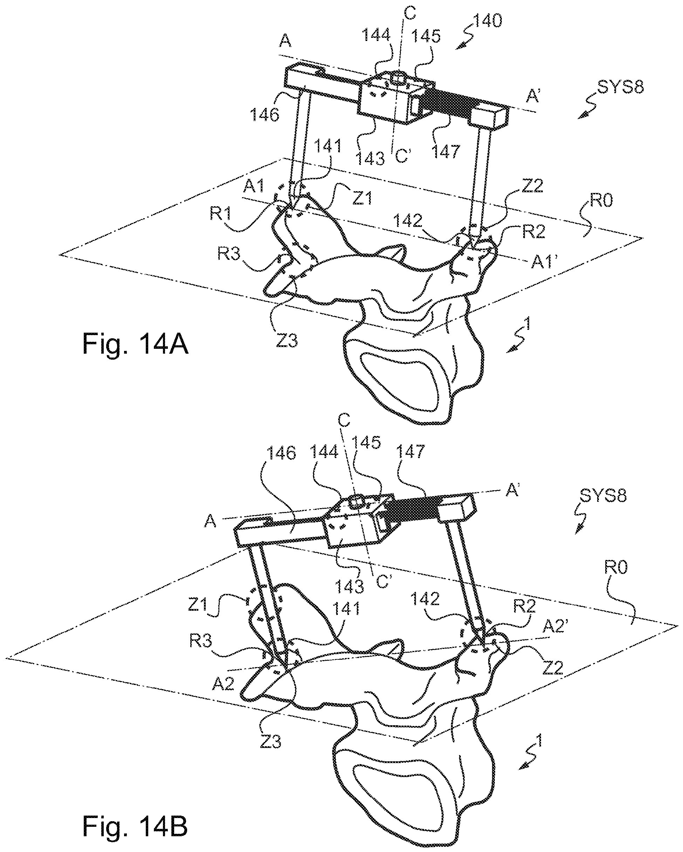

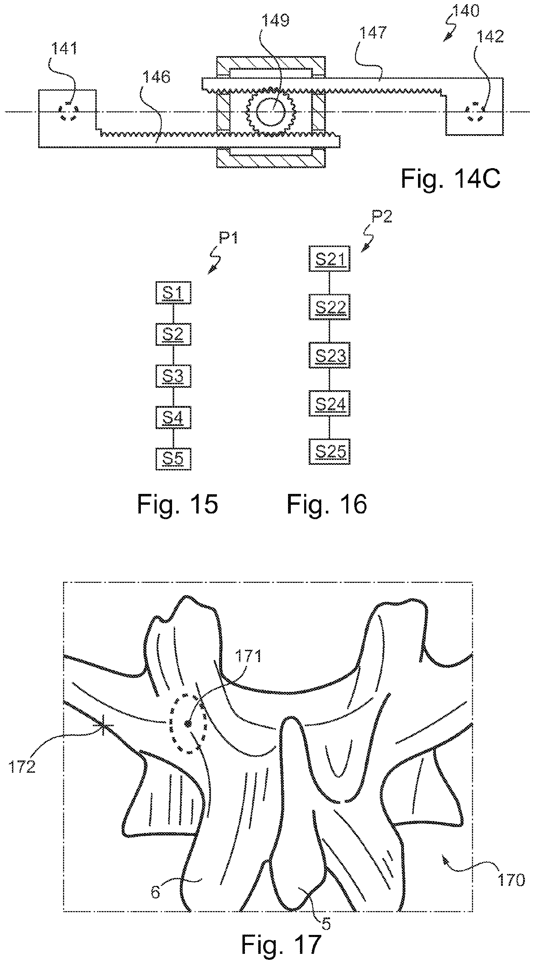

Les **figures 14A** et **14B** représentent des vues en perspective d'un système d'orientation simplifié SYS8 selon un autre exemple ne faisant pas partie de l'invention, dans deux positions différentes de mesure. La **figure 14C** représente une vue du dessus du système SYS8.

Le système SYS8 comprend un ancillaire 140 et un outil (non montré pour des raisons de simplicité).

A l'instar de l'ancillaire 130, l'ancillaire 140 est en forme de compas à deux branches 146, 147, et comporte deux extrémités terminales de contact 141, 142 destinée à venir en contact avec les zones de référence Z1, Z2, Z3 ; une partie fixe ou « carter » 143 relativement à laquelle les deux branches 146, 147 se déplacent pour ouvrir ou fermer l'ancillaire 140, de sorte que, lors de l'ouverture ou de la fermeture de l'ancillaire, les première et deuxième extrémités terminales 141, 142 forment un axe (A1-A1') constamment parallèle à un axe de référence (A-A') de la partie fixe 143. L'ancillaire 140 comprend en outre un moyen de détermination 144 d'une orientation de l'ancillaire dans un référentiel galiléen (éventuellement au sein d'un moyen de détermination d'un référentiel d'orientation ou d'intervention RA), type centrale inertielle décrite ci-dessus. L'ancillaire 140 comprend, optionnellement, un moyen de communication 145 des orientations déterminées (non représenté en détail). Ces moyens 134, 135 peuvent éventuellement être prévus amovibles comme dans la **figure 6.**

L'ancillaire 140 se distingue de l'ancillaire 130 en ce que les première et deuxième branches 146, 147 sont montées mobiles en translation par rapport à la partie fixe et sont agencées pour coulisser en translation, éventuellement de façon symétrique, relativement à la partie fixe 143. Le déplacement en translation des branches 136, 137 de l'ancillaire peut être ajusté en fonction de l'écartement entre les zones de référence Z1, Z2, pour s'adapter, avec un ancillaire unique, aux dimensions fortement variables de plusieurs zones d'intervention.

Dans cet exemple, les longueurs des branches 146, 147 peuvent être ajustées selon l'axe A-A' à l'aide d'un pignon 149 qui s'engage dans des rails d'ajustement pourvus sur les branches 146, 147 (**figure 14C**).

Dans cet exemple, les extrémités terminales 141, 142 sont des pointes. En variante, les configurations exposées ci-dessus aux **figures 1** à **12** peuvent être utilisées.

Dans l'exemple de la figure, le moyen de détermination 134 est de type centrale inertielle montée solidaire de la partie fixe. En variante, une telle centrale inertielle peut être montée solidaire de la première ou deuxième branche 146, 147.

L'ancillaire 140 s'utilise de façon similaire à l'ancillaire 130 pour déterminer *in situ* un référentiel d'intervention d'une zone d'intervention, dans un référentiel d'orientation galiléen. Pour ce faire, les deux extrémités terminales 141, 142 de l'ancillaire 140 sont positionnées successivement sur chacune de deux paires de zones de référence (Z1 et Z2 sur la **figure 14A,** et Z2 et Z3 sur la **figure 14B**), permettant d'obtenir les deux orientations non parallèles (A1-A1') et (A2-A2').

Les systèmes d'orientation simplifiés SYS7 et SYS8 peuvent être tout d'abord utilisés pour des interventions chirurgicales, selon un processus assez similaire à celui des **figures 10** et **11**. Ils peuvent également être utilisés pour intervenir avec précision sur des pièces mécaniques, hors contexte chirurgical, par exemple pour percer une pièce ou visser un élément dans une pièce selon un axe précis (parfois avec une orientation désirée selon un deuxième axe).

Un procédé de guidage pour une intervention sur une zone d'intervention comprend alors une phase préalable visant à obtenir au moins trois zones de référence dans la zone d'intervention ; à obtenir un référentiel d'intervention au moyen des zones de référence ; et à déterminer au moins un axe d'intervention (voire deux) dans le référentiel d'intervention, pour un geste d'intervention à réaliser. Ce procédé est illustré par les **figures 15** et **16.**

Cette phase préalable P1 illustrée à la **figure 15** peut être similaire à celle de la **figure 10,** consistant à obtenir, par tout moyen, une modélisation tridimensionnelle de la pièce sur laquelle procéder l'intervention (étape S1). Trois zones de référence Z1, Z2, Z3, préférentiellement trois points de référence R1, R2, R3, sont alors déterminées, par exemple par un opérateur (étape S2). Le référentiel d'intervention est alors calculé à partir des trois points de référence (étape S3), puis au moins un vecteur directeur [V]ro (voire deux) est déterminé pour le geste d'intervention à réaliser (étape S4). A l'étape S5, le procédé est répété si nécessaire pour d'autres zones d'intervention.

Suite à la phase préalable, l'intervention consiste à déterminer *in situ* le référentiel d'intervention de la zone d'intervention dans un référentiel d'orientation galiléen dans lequel se situe l'opérateur, puis à réaliser le geste d'intervention sur la zone d'intervention.

De façon assez similaire à la **figure 11,** cette phase d'intervention P2 illustrée à la **figure 16** peut comprendre l'accès à la zone d'intervention (étape S21).

Puis, l'ancillaire 130, 140 est posé sur la zone d'intervention 1 pour une première mesure. Les extrémités 131, 132, 141, 142 sont posées sur les zones de références Z1, Z2. Un axe (A1-A1') est formé entre les deux extrémités. Si nécessaire, la distance entre les extrémités 141, 142 est modifiée par le pignon 149 ou la distance entre les extrémités 131, 132 est modifiée par ouverture/fermeture du compas 130. Cette position de mesure est illustrée aux **figures 13A** et **14A.**

Après stabilisation de l'ancillaire, une mesure de l'orientation de l'axe (A1-A1') est réalisée par la centrale inertielle 134, 144 (étape S22). En variante, un bouton poussoir (non représenté) peut être prévu sur le carter 133, 143 de l'ancillaire pour permettre à l'opérateur de déclencher la mesure à la demande.

Une première orientation du référentiel d'intervention est ainsi obtenue.

Puis, l'ancillaire 130, 140 est déplacé sur la zone d'intervention 1 pour une deuxième mesure. Les extrémités 131, 132, 141, 142 sont posées sur une autre paire de zones de références, ici Z2, Z3 (il pourrait s'agir de deux zones différentes de celles utilisées pour la première mesure). Un axe (A2-A2') est formé entre les deux extrémités. Si nécessaire, la distance entre les extrémités 141, 142 est modifiée par le pignon 149 ou la distance entre les extrémités 131, 132 est modifiée par ouverture/fermeture du compas 130. Cette position de mesure est illustrée aux **figures 13B** et **14B.**

Après stabilisation de l'ancillaire ou appui sur le bouton poussoir, une mesure de l'orientation de l'axe (A2-A2') est réalisée par la centrale inertielle 134, 144 (étape S23). Une deuxième orientation, non parallèle à la première, du référentiel d'orientation est ainsi obtenue.

A l'aide de ces deux orientations mesurées, le référentiel d'intervention de la zone d'intervention est déterminé par exemple par les moyens de détermination 134, 144 et communiqué à l'extérieur de l'ancillaire 130, 140 par les moyens de communication 135, 145 (étape S24). Cette détermination permet alors, à l'aide de l'équation 1, le calcul de la matrice de rotation Mrors et du vecteur directeur [V]rs du geste à réaliser dans le référentiel galiléen.

Une fois le référentiel d'intervention dans le référentiel galiléen déterminé et communiqué, l'opérateur effectue le geste d'intervention prévu sur la zone d'intervention à l'aide d'un outil d'intervention, par exemple il procède au perçage de trous à l'aide de l'outil (étape S25). L'outil d'intervention peut être du type celui représenté en **figure 2A** ou **6.**

Pour ce faire, l'axe O-O' (non représenté) de l'outil d'intervention dans le référentiel d'orientation galiléen est mis en correspondance avec l'axe d'intervention dans le référentiel d'intervention, par exemple par conversion d'au moins un desdits axes d'un référentiel à l'autre (par exemple avec la matrice de rotation susmentionnée).

Préférablement, l'outil d'intervention comporte des moyens de détermination MEMS 34,84 et de communication 35,85 filaire ou non-filaire (sans contact), permettant de déterminer une orientation (ou axe) de l'outil en temps réel dans le référentiel d'orientation galiléen et la transmettre à l'extérieur. Ainsi, l'opérateur peut être guidé efficacement pour aligner son outil avec l'axe d'intervention prédéterminé.

Par exemple comme illustré sur la **figure 17,** un modèle numérique tridimensionnel 170 de la zone d'intervention 1 est affiché à l'opérateur, sur un écran 111 selon l'axe d'intervention. En d'autres termes, l'image affichée correspond à une vue dans un plan perpendiculaire à l'axe d'intervention, de sorte qu'un point d'intervention et l'axe d'intervention soient confondus sur l'écran.

Ainsi, un indicateur 171 (par exemple une croix ou un rond) du point d'intervention sur la zone d'intervention et un indicateur d'orientation 172, dans le référentiel d'affichage, de l'outil d'intervention positionné sur le point d'intervention peuvent être affichés sur l'écran. Pour obtenir l'indicateur 172 d'orientation de l'outil, un décalage angulaire entre l'axe d'intervention et l'axe de l'outil peut être estimé, en comparant les deux orientations associées dans le même référentiel, par exemple le vecteur directeur [V]rs à un vecteur directeur de l'axe O-O' dans le référentiel galiléen. Ce décalage angulaire peut être proportionnellement retranscrit sur l'écran au travers d'un décalage spatial de l'indicateur d'orientation 172 par rapport au point d'intervention 171, dans la direction de décalage compte tenu du référentiel d'affichage.

Dès lors, l'opérateur cherche à ajuster l'inclinaison de son outil pour faire se confondre les deux indicateurs. Avantageusement, l'opérateur peut disposer l'extrémité 32 de son outil 30 sur le point 171 pour aligner plus facilement l'outil selon l'axe d'intervention désiré (Vrs), en ne déplaçant que la poignée (33, 81) de l'outil 30 ou 70. Puis, il réalise le geste d'intervention sur la zone d'intervention lorsque les deux indicateurs 171, 172 sont confondus.

Bien entendu, il peut être prévu plusieurs axes d'intervention définissant une orientation plus précise de l'outil dans le référentiel d'intervention. Dans ce cas, l'affichage peut prévoir deux indicateurs représentatifs des deux axes d'intervention souhaités, et deux indicateurs représentatifs des axes correspondants de l'outil. Ainsi, l'opérateur manipule son outil de sorte à faire correspondre les deux paires d'indicateurs.

Par ailleurs, bien que les ancillaires 130, 140 ont été principalement décrits avec un moyen de détermination 134, 144 de type centrale inertielle, d'autres moyens de détermination d'une ou plusieurs orientations de l'ancillaire peuvent être prévus en variante. A titre d'exemple, l'ancillaire de type compas peut être équipé, à l'instar du mode de réalisation de la **figure 5,** de marqueurs optiques non-alignés et destinés à être captés par une pluralité de caméras qui filment la salle d'intervention en temps réel, afin de repérer les positions, et donc les orientations, des ancillaires dans le référentiel galiléen. Par exemple, un marqueur optique peut être porté par le carter 133,143, et un marqueur optique peut être prévu sur chacune des branches 136,137 et 146,147, par exemple dans une zone voisine des extrémités terminales 131,132 et 141,142.

L'ancillaire de type compas 130, 140 peut être fourni dans un kit comprenant également un ancillaire en Y décrit ci-dessus et un outil d'intervention. Préférentiellement, une partie amovible 60-2 comportant le moyen de détermination du référentiel d'intervention et le moyen de communication peut être prévue, qui se fixe temporairement et à la demande sur l'un des ancillaires et outil du kit, à l'instar de la **figure 6.**

## Revendications

1. Ancillaire (20 ; 20' ; 50 ; 60 ; 90 ; 200) chirurgical en forme de i-grec (Y) comprenant au moins :

- une première partie de contact (21 ; 51 ; 61 ; 91 ; 210) destinée à venir en contact avec une première zone de référence (Z1) d'une zone opératoire (ZO) ;
- une deuxième partie de contact (22 ; 52 ; 62 ; 92 ; 220) destinée à venir en contact avec une deuxième zone de référence (Z2) de la zone opératoire ;
- au moins deux branches (26, 27 ; 56, 57 ; 66, 67 ; 96, 97 ; 260 ; 270) aux extrémités desquelles les première et deuxième parties de contact (21, 22 ; 51, 52 ; 61, 62 ; 91, 92 ; 260, 270) sont disposées ;
- une troisième partie de contact (23 ; 53 ; 63 ; 93 ; 230) destinée à venir en contact avec une troisième zone de référence (Z3 ; R3) de la zone opératoire ;

- une troisième branche (28 ; 58 ; 68 ; 98 ; 280) en forme de poignée ;
- une zone centrale ayant une face inférieure qui forme la troisième partie de contact (23 ; 53 ; 63 ; 93 ; 230) ; et
- un moyen de détermination (24 ; 54A, 54B, 54C ; 84 ; 94) d'un référentiel d'orientation (RA) de l'ancillaire dans un référentiel d'orientation galiléen (RS),

**caractérisé en ce qu'**au moins une des première et deuxième parties de contact comporte une extrémité de section courbe concave ou convexe destinée à venir en contact avec au moins deux points de référence (R1 ; R1' ; R2 ; R2') dans la première ou deuxième zone de référence (Z1 ; Z2) correspondante qui est de forme convexe ou concave respectivement.

2. Ancillaire (20 ; 20' ; 50 ; 60 ; 90 ; 200) selon la revendication 1, dans lequel les première et deuxième parties de contact comportent chacune une extrémité concave ou convexe destinée à venir en contact avec au moins deux points de référence de la zone de référence convexe ou concave correspondante.

3. Ancillaire (20 ; 20' ; 50 ; 60 ; 90 ; 200) selon la revendication 2, dans lequel les deux extrémités sont de section courbe avec des axes générateurs ($\Delta$) sensiblement coaxiaux.

4. Ancillaire (20 ; 20' ; 50 ; 60 ; 90 ; 200) selon la revendication 2, dans lequel les deux extrémités présentent des sections transversales courbes et présentent des sections longitudinales qui appartiennent à une même courbe conique.

5. Ancillaire (20 ; 20' ; 50 ; 60 ; 90 ; 200) selon l'une des revendications 1 à 4, dans lequel l'extrémité convexe comporte un élément en forme de rouleau, galet, cylindre ou sphère, destiné à s'engager dans la zone de référence concave correspondante, ou l'extrémité concave comporte un élément en forme de gouttière ou de coupelle sphérique, destiné à s'engager sur la zone de référence convexe correspondante.

6. Ancillaire (20 ; 20' ; 50 ; 60 ; 90 ; 200) selon la revendication 5, dans lequel l'élément formant extrémité convexe ou concave est monté mobile en rotation sur l'ancillaire.

7. Ancillaire (20 ; 20' ; 50 ; 60 ; 90 ; 200) selon l'une quelconque des revendication 1 à 6, dans lequel la troisième partie de contact (23 ; 53 ; 63 ; 93 ; 230) est destinée à venir en contact avec la troisième zone de référence (Z3) par basculement de l'ancillaire alors que les première et deuxième parties de contact sont en contact avec les première et deuxième zones de référence respectivement.

8. Ancillaire (20; 20'; 50 ; 60 ; 90 ; 200) selon la revendication 7, dans lequel l'extrémité concave ou convexe comporte une surface de section courbe autour d'un axe de révolution, et le basculement s'opère autour de l'axe de révolution.

9. Système d'orientation chirurgical (SYS1, SYS1' ; SYS2 ; SYS3 ; SYS4 ; SYS5 ; SYS6) comprenant au moins un ancillaire (20, 20' ; 50 ; 60 ; 90 ; 200) selon l'une des revendications 1 à 8 et un outil chirurgical (30 ; 70) comprenant :

   - un moyen de détermination (34 ; 84) d'un référentiel d'orientation (RT) de l'outil ; et
   - un moyen de communication (35 ; 85) du référentiel d'orientation (RT) de l'outil.

10. Système (SYS4) selon la revendication 9, dans lequel l'ancillaire (60-1) et l'outil chirurgical (70) sont configurés pour être couplés à un même dispositif (60-2) de détermination et de communication de référentiel d'orientation (RA, RT).

11. Salle opératoire (ZS) équipée d'un ancillaire (20, 20' ; 50 ; 60 ; 90 ; 200) chirurgical selon l'une des revendications 1 à 8 et d'un dispositif d'affichage d'images et de traitement de données (110) comprenant :

   - un écran (111) pour afficher des images (I) prises de la zone opératoire (ZO) ;
   - un processeur (112) ;
   - des moyens d'entrée et de manipulation de données (113) ; et
   - des moyens de réception (114) des données communiquées par l'ancillaire.

**Patentansprüche**

1. Chirurgisches Hilfsinstrument (20; 20'; 50; 60; 90; 200) mit der Form eines Ypsilon (Y), mindestens umfassend:

   - einen ersten Kontaktteil (21; 51; 61; 91; 210), der dazu bestimmt ist, in Kontakt mit einem ersten Referenzbereich (Z1) eines Operationsbereichs (ZO) zu kommen;
   - einen zweiten Kontaktteil (22; 52; 62; 92; 220), der dazu bestimmt ist, in Kontakt mit einem zweiten Referenzbereich (Z2) des Operationsbereichs zu kommen;
   - mindestens zwei Schenkel (26, 27; 56, 57; 66, 67; 96, 97; 260; 270), an deren Enden der erste und der zweite Kontaktteil (21, 22; 51, 52; 61, 62; 91, 92; 260; 270) angeordnet sind;
   - einen dritten Kontaktteil (23; 53; 63; 93; 230), der dazu bestimmt ist, in Kontakt mit einem dritten Referenzbereich (Z3; R3) des Operationsbereichs zu kommen;
   - einen dritten Schenkel (28; 58; 68; 98; 280) in der Form eines Griffs;
   - einen zentralen Bereich, der eine Innenfläche aufweist, die den dritten Kontaktteil (23; 53; 63; 93; 230) bildet; und
   - ein Mittel zum Bestimmen (24; 54A, 54B, 54C; 84; 94) eines Orientierungsreferenzrahmens (RA) des Hilfsinstruments in einem galileischen Orientierungsreferenzrahmen (RS),

   **dadurch gekennzeichnet, dass** mindestens einer des ersten und des zweiten Kontaktteils ein Ende mit konkav oder konvex gekrümmtem Querschnitt aufweist, das dazu bestimmt ist, in Kontakt mit mindestens zwei Referenzpunkten (R1; R1'; R2; R2') in dem entsprechenden ersten oder zweiten Referenzbereich (Z1; Z2) zu kommen, der konvex bzw. konkav geformt ist.

2. Hilfsinstrument (20; 20'; 50; 60; 90; 200) nach Anspruch 1, wobei der erste und der zweite Kontaktteil jeweils ein konkaves oder konvexes Ende aufweisen, das dazu bestimmt ist, in Kontakt mit mindestens zwei Referenzpunkten dem entsprechenden konvexen oder konkaven Referenzbereich zu kommen.

3. Hilfsinstrument (20; 20'; 50; 60; 90; 200) nach Anspruch 2, wobei die beiden Enden einen gekrümmten Querschnitt mit im Wesentlichen koaxialen Erzeugendenachsen (Δ) haben.

4. Hilfsinstrument (20; 20'; 50; 60; 90; 200) nach Anspruch 2, wobei die beiden Enden gekrümmte Querschnittsflächen und Längsquerschnitte aufweisen, die zu einer einzigen konischen Kurve gehören.

5. Hilfsinstrument (20; 20'; 50; 60; 90; 200) nach einem der Ansprüche 1 bis 4, wobei das konvexe Ende ein Element in der Form einer Rolle, Walze, eines Zylinders oder einer Kugel aufweist, das dazu bestimmt ist, in den entsprechenden konkaven Referenzbereich einzugreifen, oder das konkave Ende ein Element in der Form einer Rinne oder einer kugelförmigen Schale aufweist, das dazu bestimmt ist, in den entsprechenden konvexen Referenzbereich einzugreifen.

6. Hilfsinstrument (20; 20'; 50; 60; 90; 200) nach Anspruch 5, wobei das Element, das ein konvexes oder konkaves Ende bildet, drehbar auf dem Hilfsinstrument angebracht ist.

7. Hilfsinstrument (20; 20'; 50; 60; 90; 200) nach einem der Ansprüche 1 bis 6, wobei der dritte Kontaktteil (23; 53; 63; 93; 230) dazu bestimmt ist, in Kontakt mit dem dritten Referenzbereich (Z3) durch ein Kippen des Hilfsinstruments zu kommen, während das erste und das zweite Kontaktteil mit dem ersten bzw. dem zweiten Referenzfläche in Kontakt sind.

8. Hilfsinstrument (20; 20'; 50; 60; 90; 200) nach Anspruch 7, wobei das konkave oder konvexe Ende eine Fläche mit einem gekrümmten Querschnitt um eine Drehachse aufweist und das Kippen um die Drehachse erfolgt.

9. Chirurgisches Orientierungssystem (SYS1, SYS1'; SYS2; SYS3; SYS4; SYS5; SYS6), umfassend mindestens ein Hilfsinstrument (20; 20'; 50; 60; 90; 200) nach einem der Ansprüche 1 bis 8 und ein chirurgisches Werkzeug (30; 70), umfassend:

   - ein Mittel zum Bestimmen (34; 84) eines Orientierungsreferenzrahmens (RT) des Werkzeugs; und
   - ein Mittel zum Übermitteln (35; 85) des Orientierungsreferenzrahmens (RT) des Werkzeugs.

10. System (SYS4) nach Anspruch 9, wobei das Hilfsinstrument (60-1) und das chirurgische Werkzeug (70) dazu konfiguriert sind, mit einer einzigen Vorrichtung (60-2) zum Bestimmen und Übermitteln des Orientierungsreferenzrahmens (RA, RT) gekoppelt zu werden.

11. Operationssaal (ZS), der mit einem chirurgischen Hilfsinstrument (20; 20'; 50; 60; 90; 200) nach einem der Ansprüche 1 bis 8 und einer Bildanzeige- und Datenverarbeitungsvorrichtung (110) ausgestattet ist, umfassend:

- einen Bildschirm (111) zum Anzeigen von Bildern (I), die in dem Operationsbereich (ZO) aufgenommen werden;
- einen Prozessor (112);
- ein Mittel zum Eingeben und Bearbeiten von Daten (113); und
- ein Mittel (114) zum Empfangen von Daten, die von dem Hilfsinstrument übermittelt werden.

## Claims

1. An ancillary surgical instrument (20 ; 20'; 50 ; 60 ; 90 ; 200) in the shape of a letter wye (Y) comprising at least:

- a first contact part (21; 51 ; 61 ; 91 ; 210) configured to come into contact with a first reference area (Z1) of an operating area (ZO);
- a second contact part (22 ; 52 ; 62 ; 92 ; 220) configured to come into contact with a second reference area (Z2) of the operating area;
- at least two branches (26, 27 ; 56, 57 ; 66, 67 ; 96, 97 ; 260 ; 270) at the ends of which the first and second contact parts (21, 22 ; 51, 52 ; 61, 62 ; 91, 92 ; 260, 270) are disposed;
- a third contact part (23 ; 53 ; 63 ; 93 ; 230) configured to come into contact with a third reference area (Z3 ; R3) of the operating area;
- a third branch (28 ; 58 ; 68 ; 98 ; 280) in the form of a handle;
- a central area having a lower face which forms the third contact part (23 ; 53 ; 63 ; 93 ; 230); and
- a means (24 ; 54A, 54B, 54C ; 84 ; 94) for determining an orientation reference frame (RA) of the ancillary instrument in a Galilean orientation reference frame (RS),

**characterized in that** at least one of the first and second contact parts comprises a concave or convex curved cross-section end configured to come into contact with at least two reference points (R1 ; R1' ; R2 ; R2') in the first or second corresponding reference area (Z1 ; Z2) which is of convex or concave shape respectively.

2. The ancillary instrument (20 ; 20'; 50 ; 60 ; 90 ; 200) according to claim 1, wherein the first and second contact parts each comprise a concave or convex end configured to come into contact with at least two reference points of the corresponding convex or concave reference area.

3. The ancillary instrument (20 ; 20'; 50 ; 60 ; 90 ; 200) according to claim 2, wherein the two ends are of curved cross-section with substantially coaxial generatrices ($\Delta$).

4. The ancillary instrument (20 ; 20'; 50; 60 ; 90 ; 200) according to claim 2, wherein the two ends have curved cross-sections and have longitudinal cross-sections which belong to the same conic section.

5. The ancillary instrument (20 ; 20' ; 50 ; 60 ; 90 ; 200) according to one of claims 1 to 4, wherein the convex end comprises a member in the form of a roll, roller, cylinder or sphere configured to engage in the corresponding concave reference area, or the concave end comprises a channel-shaped member or spherical dome, configured to engage on the corresponding convex reference area.

6. The ancillary instrument (20 ; 20'; 50 ; 60 ; 90 ; 200) according to claim 5, wherein the member forming a convex or concave end is rotatably mounted on the ancillary instrument.

7. The ancillary instrument (20 ; 20'; 50 ; 60 ; 90 ; 200) according to any one of claims 1 to 6, wherein the third contact part (23 ; 53 ; 63 ; 93 ; 230) is configured to come into contact with the third reference area (Z3) by rocking of the ancillary instrument while the first and second contact parts are in contact with the first and second reference areas respectively.

8. The ancillary instrument (20 ; 20' ; 50 ; 60 ; 90 ; 200) according to claim 7, wherein the concave or convex end comprises a surface which has a curved cross-section around an axis of revolution, and the rocking takes place around the axis of revolution.

9. A surgical orientation system (SYS1, SYS1' ; SYS2 ; SYS3 ; SYS4 ; SYS5 ; SYS6) comprising at least one ancillary instrument (20, 20'; 50 ; 60 ; 90 ; 200) according to any one of claims 1 to 8 and a surgical tool (30 ; 70) comprising:

   - means (34 ; 84) for determining an orientation reference frame (RT) of the tool; and
   - means (35 ; 85) for communicating the orientation reference frame (RT) of the tool.

10. The system (SYS4) according to claim 9, wherein the ancillary instrument (60-1) and the surgical tool (70) are configured to be coupled to one and the same device (60-2) for determining and communicating an orientation reference frame (RA, RT).

11. An operating theatre (ZS) equipped with an ancillary surgical instrument (20, 20' ; 50 ; 60 ; 90 ; 200) according to one of claims 1 to 8 and with a device (110) for displaying images and data processing comprising:

   - a screen (111) for displaying images (I) taken of the operating area (ZO);
   - a processor (112);
   - means for entering and manipulating data (113); and
   - means (114) for receiving data communicated by the ancillary instrument.

Fig. 1A

Fig. 1B

Fig. 2A

Fig. 2B

Fig. 3A

Fig. 3B

Fig. 4

Fig. 6

Fig. 5

Fig. 7

Fig. 8A

Fig. 8B

FIG.9

FIG.10

FIG.11

FIG.12

Fig. 13A

Fig. 13B

Fig. 14A

Fig. 14B

Fig. 14C

Fig. 15

Fig. 16

Fig. 17

Section longitudinale :
courbe conique

21

22

T

26

27

T'

23

20

Section
Transversale
T-T'

ZO

22

27

Fig. 18

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016102898 A